# EUROPEAN PATENT SPECIFICATION

(11) **EP 0 640 101 B1**
(45) Date of publication and mention of the grant of the patent: **08.12.1999**
(21) Application number: 93910594.6
(22) Date of filing: 13.04.1993
(51) Int. Cl.: C07K 7/08, C07K 14/47, A61K 38/10, A61K 38/16

(54) **METHODS AND REAGENTS FOR DIAGNOSIS OF AUTOANTIBODIES**
METHODEN AND REAGENTIEN ZUR DIAGNOSE VON AUTOANTIKÖRPERN
PROCEDES ET REACTIFS POUR LE DIAGNOSTIC D'AUTOANTICORPS

(30) Priority: 13.04.1992 US 867819
(43) Date of publication of application: 01.03.1995
(73) Proprietor: Oklahoma Medical Research Foundation, Oklahoma City Oklahoma 73104 (US)
(72) Inventor: HARLEY, John, B., Oklahoma City, OK 73103 (US)
(74) Representative: Bassett, Richard Simon
(86) International application number: US9303484
(87) International publication number: WO9321223

(56) References cited:
- EP-A- 0 313 156
- WO-A-88/09932
- WO-A-91/11718
- JOURNAL OF IMMUNOLOGY., vol.148, no.1, 1 April 1992, BALTIMORE US pages 2074 - 2079 J A JAMES & J B HARLEY 'Linear epitope mapping of an Sm B/B' polypeptide'
- CLIN. EXP. IMMUNOL., vol.86, no.1, October 1991, OXFORD pages 71 - 78 S BARAKAT ET AL. 'Mapping of epitopes on U1 snRNP polypeptide A with synthetic peptides and autoimmune sera'
- CHEMICAL ABSTRACTS, vol. 116, no. 1, 6 January 1992, Columbus, Ohio, US; abstract no. 4803a, Y TAKEDA ET AL. 'Antigenic domains on the U1 small nuclear ribonucleoprotein-associated 70 kD polypeptide; a comparison of regions selectively recognized by human and mouse autoantibodies and by monoclonal antibodies' page 480 ;
- Proceedings of the National Academy of Sciences, Volume 85, Number 24, issued December 1988, S.L. DEUTSCHER et al., "Molecular Analysis of the 60-kDa Human Ro Ribonucleoprotein", pages 9479-9483, see entire document.
- Journal of Clinical Investigation, Volume 83, Number 4, issued April 1989, E. BEN-CHETRIT et al., "Isolation and Characterization of a cDNA Clone Encoding the 60-kD Component of the Human SS-A/Ro Ribonucleoprotein Autoantigen", pages 1284-1292, see entire document.
- Proceedings of the National Academy of Sciences, Volume 82, Number 7, issued April 1985, J. CHAMBERS et al., "Isolation and Analysis of cDNA Clones Expressing Human Lupus La Antigen", pages 2115-2119, see entire document.
- Journal of Immunology, Volume 140, Number 9, issued 01 May 1988, A.D. STURGESS et al., "Characteristics and Epitope Mapping of a Cloned Human Autoantigen La", pages 3212-3218, see entire document.
- Journal of Biological Chemistry, Volume 263, Number 34, issued 05 December 1988, J.C. CHAMBERS et al., "Genomic Structure and Amino Acid Sequence Domains of the Human La Autoantigen", pages 18043-18051, see entire document.
- Nucleic Acids Research, Volume 17, Number 6, issued 1989, E.K.L. CHAN et al., "Ribonucleoprotein SS-B/La Belongs to a Protein Family with Consensus Sequence for RNA Binding", pages 2233-2244, see entire document.
- FEBS Letters, Volume 250, Number 2, issued 03 July 1989, N.G. SHARPE et al., "Isolation of cDNA Clones Encoding the Human Sm B/B' Autoimmune Antigen and Specifically Reacting with Human Anti-Sm Autoimmune Sera", pages 585-590, see entire document.
- Clinical Chemistry, Volume 35, Number 9, issued September 1989, M. RENZ et al., "Expression of the Major Human Ribonucleoprotein (RNP) Autoantigens of Escherichia coli and their Use in an EIA for Screening Sera from Patients with Autoimmune Diseases", pages 1861-1863, see entire document.
- EMBO Journal, Volume 8, Number 4, issued April 1989, G. McALLISTER et al., "cDNA Sequence of the Rat U snRNP-Associated Protein N: Description of a Potential Sm Epitope", pages 1177-1181, see entire document.
- Journal of Immunology, Volume 140, Number 1, issued 01 January 1988, K. YAMAMOTO et al., "Isolation and Characterization of a Complementary DNA Expressing Human U1 Small Nuclear Ribonucleoprotein C Polypeptide", pages 311-317, see entire document.
- Journal of Biological Chemistry, Volume 264, Number 9, 25 March 1989, "Primary Structure of a Human Small Nuclear Ribonucleoprotein Polypeptide as Deduced by cDNA Analysis", pages 5024-5030, see entire document.
- Journal of Immunology, Volume 145, Number 2, issued 15 July 1990, K.B. ELKON et al., "Epitope Mapping of Recombinant HeLa SmB and B' Peptides Obtained by the Polymerase Chain Reaction", pages 636-643, see entire document.
- EMBO Journal, Volume 6, Number 12, issued December 1987, P.T.G. SILLEKENS et al., "cDNA Cloning of the Human U1 snRNA-Associated A Protein: Extensive Homology Between U1 and U2 snRNP-Specific Proteins", pages 3841-3848, see entire document.

## Description

This invention is in the area of the prevention, diagnosis and treatment of autoimmune diseases, especially systemic lupus erythematosus.

The United States government has rights in this invention by virtue of grants from the National Institutes of Health AR39577, AI24717, AI21568, AI31584 and AR01844, and the Veteran's Administration.

Systemic lupus erythematosus (SLE) is similar to many other disorders in which autoantibodies are found and thought to be important in etiology and pathogenesis. SLE can be grouped with those diseases that commonly have autoantibodies present but for whom a central role of autoantibody in pathogenesis leading to clinical expression has yet to be fully established or accepted. Other such diseases include Sjogren's syndrome, rheumatoid arthritis, juvenile onset diabetes mellitus, primary biliary cirrhosis, Wegener's granulomatosis, inflammatory bowel disease, and many others.

Typically, autoimmune diseases present with a wide array of symptoms and clinical signs. The production of circulating autoantibodies to ribonucleoprotein complexes (RNPs) is a unifying characteristic of some of the rheumatic autoimmune diseases. The most common antigens in SLE and closely related disorders include: Ro/SSA, La/SSB, nRNP and Sm. Initially, these antibodies were found using double immunodiffusion, but more recently sensitive solid phase assays have been developed to quantitate the autoantibodies.

The Ro/SSA RNA-protein particle has been found to be a constituent of all human cells evaluated to date. Approximately half of Sjogren's syndrome (SS) and systemic lupus erythematosus (SLE) patients have anti-Ro/SSA precipitins. Approximately 75% of patients with subacute cutaneous lupus erythematosus or complement component C2 deficiency with SLE have anti-Ro/SSA precipitins. Over 80% of mothers of newborns with neonatal lupus dermatitis or complete congenital heart block have this autoantibody. As many as 5 % of patients with rheumatoid arthritis, polymyositis, and progressive systemic sclerosis have anti-Ro/SSA, as reported by R.M. Bernstein, et al., Mol. Biol. Med. 2:105-120 (1984); and J.B. Harley and K.K. Gaither, Autoantibodies. In Rheumatic Disease Clinics of North American: Systemic Lupus Erythematosus 14:1, 43-56 (1988).

Autoantibodies to the La/SSB ribonucleoprotein antigen are also found in patients with SS and SLE, as reported by Alspaugh, et al., Arthritis Rheum. 19:216 (1976) and Mattioli, et al., Arthritis Rheum. 17:421 (1974). In addition, these antibodies as reported by Horsfall, et al., J. Autoimmunity 4:165 (1991), thought to be pathogenic to the fetus during pregnancy in some mothers who have anti-La/SSB autoantibodies, where they are associated, along with anti-Ro/SSA, with complete congenital heart block (CCHB).

It has been an issue of intensive debate as to whether the many autoantibodies found in systemic lupus erythematosus and related diseases represent an antigen specific or a polyclonal, antigen non-specific response. Evidence that autoantibodies are important in the expression of SLE and related syndromes is convincing. Specific depletion in a heart block neonate (Harley, J.B., et al., Arthritis Rheum. 28:1321-1325 (1985)) and specific anti-Ro/SSA immunoglobin deposition in human skin (Lee, L.A., et al., J. Clin. Invest. 83:1556-1562 (1989)) have been demonstrated. Specific concentration of anti-Ro/SSA has been shown in the immunoglobulin of renal eluates from kidneys affected by lupus nephritis (Maddison, P.J. and Reichlin, M. Arthritis Rheum. 22:858-863 (1979)). Anti-Ro/SSA has been found to be specifically concentrated in a parotid gland of a patient with Sjogren's syndrome and primary biliary cirrhosis (Penner, E. and Reichlin, M. Arthritis Rheum. 25:1250-1253 (1982)). Observations that infants with transplacentally acquired maternal IgG develop neonatal lupus dermatitis and/or complete congenital heart block (Harley, J.B. and Gaither, K.K.: Autoantibodies. In Rheumatic Disease Clinics of North America: Systemic Lupus Erythematosus 14:1, 43-56 (1988)) strongly suggests that maternal autoantibody (anti-Ro/SSA or anti-La/SSB) transported across the placenta is a critical component required, but not sufficient, for these clinical problems.

The Ro/SSA family of proteins has now been shown to have several molecular forms which are operationally defined by the molecular weight of the antigen identified. A major form has an apparent molecular weight of 60 kiloDaltons (kD). This protein is associated with one of four hY RNAs. Recently, two additional proteins bound by anti-Ro/SSA sera have been identified by M.D. Rader, et al., J. Clin. Invest. 83:1556-1562 (1989), with molecular weights of 52 kD and 54 kD. A 48 kD protein, calmodulin, has been identified as being bound by anti-Ro/SSA sera (McCauliffe, et al., J. Clin. Invest. 85:1379-1391 (1990)). The La/SSB protein, a 48 kD peptide, as described by J.C. Chambers and J.D. Keene, Proc. Natl. Acad. Sci. USA 82:2115-2119 (1985), is also a member of this group of autoantibodies, and binds small RNAs with a polyuridine terminus, as reported by J.E. Stephano, Cell 36:145-154 (1984). La/SSB is bound by a third of the anti-Ro/SSA precipitin positive sera. The La/SSB protein has been purified from a variety of tissue sources and shown to be a 46 to 50 kD monomeric phosphoprotein, as reported by Habets, et al., EMBO J. 2:1625 (1983) and Venables, et al., Clin. Exp. Immunol. 54:731 (1983). It associates with RNA polymerase III transcripts, as reported by Lerner, et al., Proc. Natl. Acad. Sci. USA. 76:5495 (1979) and Steitz, et al., Cold Spring Harbor Symposium Quant. Biol. 47:893 (1983), and may function as a termination factor for this enzyme, as reported by Gottlieb, et al., EMBO J. 8:841 (1989). A nucleic acid dependent ATPase/dATPase enzymatic activity has also been attributed to La/SSB by Bachmann, et al., Cell 60:85 (1990).

Anti-Sm antibodies are frequently associated with SLE. These autoantibodies precipitate snRNP containing the U1, U2, U4/U6 and U5 RNA. These complexes form the spliceosome and splice heterogenous nuclear RNA, as reported by Sharp, Science 235:766 (1987) and Maniatis and Reed, Nature 325:673 (1987). Anti-Sm antibodies are directed against one or a combination of six polypeptides: B (26 kDa), B' (27 kDa), D (13 kDa), E/F (11 kDa doublet) and G (less than 10 kDa).

Nearly all rheumatic disease patients who form an anti-Sm precipitin in Ouchterlony immunodiffusion have or eventually develop an anti-nRNP precipitin, as reported by Fisher, et al., Arthritis Rheum. 28:1348 (1985). Anti-Sm and anti-nRNP precipitins form a line of partial identify in Ouchterlony immunodiffusion, as discussed by Mattioli and Reichlin, J. Immunol. 110:1318 (1973). The basis for this partially shared antigenicity is explained by the composition of the U snRNP particles. The antigen for the anti-nRNP precipitin are the 70 kD, A, and C peptides that are unique to the U1 snRNP, B/B' and D peptides are also found on the U1 snRNP. The B/B' and D Ag, but not the 70 kDa, A or C, are found in the U2, U4/U6 and U5 snRNP. Hence, both anti-Sm and anti-nRNP bind anti-U1 snRNP activity, but only anti-Sm binds U2, U4/U6, and U5 snRNP.

U.S. Serial No. 07/648,205 filed January 31, 1991 by John B. Harley for "Assays and Treatments for Autoimmune Diseases", and U.S. Serial No. 07/472,947 entitled "Assays and Treatments for Autoimmune Diseases" filed January 31, 1990, correspond to WO-91/11718 and describe a specific method to identify the etiologic or antigenic agent responsible for the production of autoantibodies characteristic of a particular disorder. The antigen is first isolated, using, for example, autoantibodies isolated from one or more patients. The antigen is then divided into overlapping short amino acid sequences, preferably twenty amino acids or less, most conveniently octapeptides. The sequences having the greatest reactivity with the autoantibodies are identified and then compared with all known amino acids sequences using the available computer data bases. The protein having the maximum number or proportion of sequences homologous to the sequences of greatest reactivity with the autoantibodies is among the likeliest candidate of the known sequenced proteins for the etiological agent or immunogen. Once the etiological agent and antigenic sequences are known, it is possible to design assays and reagents for the diagnosis and treatment of patients having either the etiological agent and/or autoantibodies.

Barakat et al., Clin. Exp. Immunol. (1991)86, 71-78 relates to an epitope mapping study in which thirteen peptides corresponding to residues 1-11, 7-37, 35-38, 56-77, 74-96, 103-135, 131-153, 149-166, 163-184, 180-205, 203-225, 224-251 and 257-282 in the sequence of snRNP UIA were synthesised.

The examples in the earlier applications used peptides derived from the sequence for the 60 kDa Ro/SSA protein and La/SSB, which were reactive with antisera from SLE and SS patients.

It is therefore an object of the present invention to provide additional diagnostic reagents for identifying and classifying individuals previously exposed to a particular immunogen or expressing autoantibodies reactive with Ro/SSA, La/SSB, nRNP, or Sm B/B' polypeptides, or the epitopes (or their immune equivalent) eliciting production of the autoantibodies.

It is a still further object of the present invention to provide methods and compositions for identifying and treating autoimmune disorders, such as Systemic Lupus Erythematosus and Sjogren's syndrome.

The invention provides a peptide forming a linear epitope for a human antibody as defined in the accompanying claims.

A number of octapeptides have been generated from the sequences encoding the 60 kDa Ro/SSA peptide, the La/SSB autoantigen, the 70 kD nuclear ribonucleoprotein (nRNP), and the Sm B/B' polypeptide, which represent linear epitopes for autoantibodies present in the sera of SLE and SS patients.

For example, the most important antigenic peptides derived from Sm B/B' are (29) GTFKAFDK, (45) CDEFRKIKPKNAKQP, (94) RVPLAGAA, (101) AGGPGVGRAAGRGVPAG, (125) AGLAGPVRGVGGPSQ, (140) QVMTPOGRGTVA, (165) PTQYPPGRGTPPPPV, (174) TPPPPVGRATPPPGI, (184) PPPGIMAP, (189) MAPPPGMRPPM, (202) PIGLPPARGTPIGMPP, (212) PIGMPPPG, (221) RPPPPGIRGPP, and (228) RGPPPPGMRPPR. Additional reactive peptides can be derived from (30) TFKAFDKHM, (83) EGPPPKDT, (88) KDTGIARV, AND (120) IPQAPAGLAG. These were determined by binding studies. PPPGMRPP is especially antigenic and is repeated three times in the sequence of B/B'. The antigenicity of other peptides was determined and include the shorter peptides PPPGMRP and PPPGMR. Substitution studies were also done. All 19 of the other common naturally occurring amino acids are substituted for an amino acid in a particular position. For example, the arginine in position six of PPPGMRPP can be substituted with: F, G, H, I, K, S, T, V, W and Y.

These peptides are useful in solid phase assays for patients characterized by the presence of these autoantibodies, and can be used to categorize patients as to the likelihood of developing certain conditions associated with SLE. The peptides are also potentially useful in treatment of these patients using immobilized peptide to remove autoantibody, to block binding of the autoantibodies with patient molecules reactive with the autoantibodies, or as a component of a vaccine.

### Brief Description of the Drawings

Figure 1 graphs the antigenic regions of Sm B/B', with the octapeptides binding with an absorbance greater than 0.50, and their surrounding octapeptides.

Figure 2 graphs the results of deletion studies on the Sm B/B' epitope PPPGMRPP: Figure 2a, shows the decrease in binding for removal of amino acids from the epitope; Figure 2b shows the effect of substitution of the sixth position arginine.

### Detailed Description of the Invention

A number of peptides, preferably consisting of eight amino acids, are disclosed that are bound by human autoantibodies characteristic of SLE and other autoimmune disorders. The peptides are made synthetically, based on the published amino acid sequences for known autoantigens, Ro/SSA, La/SSB, nRNP, and Sm B/B'. They have a variety of uses, for example, as components in diagnostic assays, potentially as therapeutics, and in research on the possible causes of these autoimmune diseases.

### Definition of Linear Epitopes and Methods of Synthesis

As used herein, a peptide is defined as consisting of less than one hundred amino acids and will generally be an octapeptide. Peptides of up to forty amino acids, more preferably of between four and twenty-five amino acids, can be synthesized using any one of the methods known to those skilled in the art. A preferred method is described in the detail in the examples. The octapeptides described in the examples herein are derived from published sequences encoding the autoantigens. The number in parenthesis is the position in the sequence for the first amino acid residue of the first octapeptide that binds. The underlined part of the sequences are the octapeptides with the greatest binding.

Although described with reference to specific sequences, a number of substitutions using natural or synthetic amino acids can be made in the peptides to yield an peptide acting as a linear epitope that is functionally equivalent to the disclosed sequence, as demonstrated by examples 3 and 4. Accordingly, the term linear epitope as defined by a specific sequence is used herein to include peptides having substitutions yielding a peptide bound in an equivalent manner or extent by an antibody.

For example, using monoclonal antibodies against peptide determinants of Sm B/B', substitution studies demonstrated that A, G, and S can substitute for R in PPPGMRPP in the binding of one antibody, KSm3. Analogously, F, H, T, V and Y can substitute for I in PPPGIRGP in the binding of KSm3.

While the monoclonal antibodies have great potential to be instructive, it is important to realize that the polyclonal autoimmune sera obtained from patients may lead to much more complicated binding phenomena.

### Screening of Peptides by Binding to Autoantibodies.

Solid phase binding of autoantibodies to peptides has proven useful for examining sequential linear epitopes and defining important residues in epitope structure but is expected to be less useful in defining conformational epitopes or regions where two or more linear, but not sequential, epitopes are brought together by the tertiary structure. In addition, although many peptides will tend to assume conformations in solution that are not found in the native protein structure, true epitopes may still be delineated by this method. Those peptides that tend to have a structure similar to that found in the native molecule will be bound by a larger proportion of the autoantibodies that bind the analogous sequence on the native protein and may even be bound with greater affinity.

The examples below describe in detail how the peptides can be synthesized and screened for binding.

### Use of Peptides in Treatment and Classification of Patients

Subsets of antigenic peptides have the potential to identify patients at risk for particular clinical manifestations or patients in particular prognostic groups. The peptides disclosed herein can be used in combination in assays, such as the solid phase assay, to classify patients.

Specifically, the peptides that are bound by autoantibodies in patients characterized by specific disorders, such as renal disease or central nervous system involvement, are selected and combined in an assay, such as an ELISA for a test to detect the collection autoantibodies that bind this particular collection of peptides. Using a mixture of peptides may increase the efficiency and reliability of such assays, as compared with using a single autoantigen, or a single peptide.

The peptides can be used in solution or immobilized to a solid substrate, such as a gel suitable for affinity chromatography, or a multi-well plate, using standard techniques such as the commercially available cyanogen bromide.

The peptides can be used therapeutically in combination with a pharmaceutically acceptable carrier. The peptides can be administered in a dosage effective to block autoantibodies or as a vaccine to block the autoantibodies, by eliciting an immune response. The peptide acts as a functional antagonist by binding to antibody that does not stimulate or activate the immune cells and thereby block the immune response to the autoantigens.

Pharmaceutical carriers are known to those skilled in the art and include encapsulation of compounds for oral administration, for example, in an enteric coating or in combination with a binder such as stearate or lactose, or in solution. Acceptable solutions include sterile water, saline, and buffered solutions at physiological pH. Peptides used as vaccines can be administered orally, intramuscularly or subcutaneously. Other compounds will be administered according to standard procedures used by those skilled in the art. As defined herein, a pharmaceutical carrier is usually inert by itself but may have biological activity. For example, a vaccine may consist of immunogenic peptides or proteins in combination with an adjuvant.

Alternatively, the peptides used for treatment might include peptides homologous to an identified antigenic sequence. These peptides, either free or bound to a carrier, could be delivered to a patient in order to decrease the amount of circulating antibody with a particular specificity. In addition, knowledge of the cross-reacting epitopes between a foreign antigen and an autoantigen may allow for re-induction of tolerance. It is well known in experimental models of the immune response that the response can be suppressed and tolerance induced by treatment with the antigen. Peptide therapy with the cross-reacting sequences may be a potential therapy in autoimmune diseases.

The amino acid sequences can also be used to make agents for neutralizing circulating antibodies or immobilized on substrates in extracorporeal devices for specific removal of autoantibodies, using methodology known to those skilled in the art.

The present invention will be further understood with reference to the following non-limiting examples:

### Example 1: Identification of Linear Epitopes of the La/SSB Autoantigen.

### Patients and Methods

Peptide Synthesis. The La/SSB amino acid sequence as predicted from the nucleotide sequence of cDNA clones was as reported by Chambers, et al., J. Biol. Chem. 263:18043 (1988) and Sturgess, et al., J. Immunol. 140:3212 (1988), the teachings of which are incorporated herein. The presumably complete 408 amino acid La/SSB peptide sequence was used to construct simultaneously sequential octapeptides, each overlapping its neighbor by seven amino acids, on polystyrene pins. The entire amino acid sequence of the La/SSB protein was synthesized on five blocks of 96 pins in an 8 X 12 format. Onto each pin block, three identical positive control octapeptides were synthesized with the sequence EYRKKMDI, which represents a major epitope from the carboxyl terminal sequence of the human 60 kD Ro/SSA protein. Incubating dilutions of anti-Ro/SSA reference serum on these control pins during each assay made comparisons possible among plates and between assays.

Solid Phase Anti-peptide Assay. All steps were carried out by immersing the pin blocks into microtiter plate wells. Pins were incubated in blocking buffer (1 % bovine serum albumin (BSA) in phosphate buffered saline (PBS), pH 7.2) for 1 h at room temperature and then in 1:100 dilutions of sera in diluent (1 % BSA and 0.05% Tween in PBS) overnight at 40°C in humidified containers. Pin blocks were washed four times with wash buffer (0.05% Tween in PBS) for 10 min with agitation and then immersed for 1 h at room temperature in affinity purified goat anti-human - chain specific antibody conjugated to alkaline phosphatase (Sigma Chemical Co., St. Louis, MO) diluted 1:1000 in diluent. After washing as above, pins were incubated in para-nitrophenyl phosphate solution at 37°C for 2 h. Color development was read at 410 nm on a Dynatech MR5000 ELISA plate reader.

Regeneration of Pins. After substrate development, blocks of pins were incubated in a 50-60°C sonicating water bath containing freshly prepared 1 % sodium dodecyl sulfate and 0.1 % 2-mercaptoethanol for 1 h. They were then rinsed twice in distilled water, pre-heated to 50-60°C, and finally immersed in boiling methanol for 2 min prior to air drying.

Expression of Results. Variation within and among assays was standardized by normalizing Ro/SSA peptide positive control pins present on each block, which had been incubated with anti-Ro/SSA reference serum at a dilution of 3:1000, to a constant value of 0.175 at A₄₁₀. All absorbance data was thus normalized by multiplication with the conversion factor A_{obs}/0.175, where A_{obs} is the observed A₄₁₀ given by the Ro/SSA control pin incubated with 3:1000 anti-Ro/SSA reference serum for any given assay. Absorbance for each octapeptide was plotted using a spreadsheet program (AOK.abc Version 2.4) on a VAX 8250/VMS computer.

Samples. Sera from five normal, healthy donors and ten patients with primary SS and/or SLE were screened by pin ELISA for antibodies binding to the La/SSB octapeptides. Eight of these ten patients had both anti-Ro/SSA and anti-La/SSB precipitin forming autoantibodies, and seven of these eight had borne children with congenital heart block (CCHB). Two patients had antibodies to Ro/SSA alone. Anti-La/SSB antibodies were affinity purified from the sera of two patients on La/SSB cross-linked immunosorbents as described by Horsfall, et al., J. Immunol. Meth. 104:43 (1987).

Assessment of Linear Sequence Epitopes. Background binding was defined by the total average reactivity (A₄₁₀) of five normal sera to the entire La/SSB sequence (O.D. = 0.333 ± 0.128 standard deviation). Regions of reactivity greater than or equal to 3.5 standard deviations above background binding (equivalent to 0.9998 of the normal distribution) and bound by at least three patient sera were taken to represent possible La/SSB epitopes. Epitopes were numbered in order from most to least reactive. Operationally, this was defined first by numbers of patients binding greater than or equal to 3.5 standard deviations above the normal mean as a measure of the degree of conservation between sera. As a secondary criterion, the average magnitude of peak reactivity among patients in the region of the putative epitope was used to rank epitopes having equal numbers of patient sera binding. In some cases an epitope could be a single octapeptide and in others a broad region of reactivity across several octapeptides was observed. In some of these latter cases, more than one putative epitope was identified.

### Results

Binding of Anti-La/SSB Antibodies to La/SSB Octapeptides. All eight anti-La/SSB positive patient sera have strongly bound selected La/SSB Octapeptides, which span the entire sequence from the amino-terminal to the carboxyl-terminal regions. Normal sera also bind, but at a much reduced level. Sera lacking anti-La/SSB antibodies, though possessing high titers of other autoantibodies, have also shown low reactivity to the La/SSB sequence, consistent with a background response. Goat anti-human gamma-chain specific antibody conjugate used in these studies does not significantly bind to the octapeptides in the absence of human serum.

Epitopes Defined by Anti-La/SSB Sera from Patients. Positive reactivity has been defined as that greater than or equal to 3.5 standard deviations above the mean reactivity of five normal sera to overlapping octapeptides from the entire La/SSB sequence. Those regions which have been recognized by three or more anti-La/SSB sera have been defined as possible epitopes in this study, although regions reactive with one or two autoimmune sera may also constitute epitopes.

No epitope has been bound by all eight sera tested; however, 13 of the 18 epitopes defined in this way have been bound by at least four sera. In addition, peak reactivity within each epitope tends to vary among the patient sera, reflecting individual responses. Thus, although three patients have bound octapeptides of epitope 18, their greatest binding has not been found to occur with the same octapeptide. Control sera also have minimal, though positive, binding to the La/SSB sequence by this criterion. More importantly, however, two other autoimmune sera with no detectable anti-La/SSB antibodies do not bind any octapeptide from the La/SSB sequence by a magnitude of 3.5 standard deviations above the mean of the normal sera.

Epitopes have been subsequently numbered from greatest to least, taking first into account the number of sera reacting, and second the relative magnitude of the responses, as shown in Table I. Some of these epitopes correspond to areas of high antigenic index or hydrophilicity as predicted by Kyte and Doolittle plots, J. Mol. Biol. 157:105 (1982).

**Table 1.**

| La/SSB autoepitopes | | |
|---|---|---|
| **Number** | **Position** | **Sequence** |
| 1 | 136-153 | QVLNIQMRRTLHKAFKGS |
| 2 | 17-37 | ICHQEYYFGDFNLPRDKFLK |
| 3 | 46-57 | WVPLEIMIKFNR |
| 4 | 86-97 | KKTKIRRSPSKPL |
| 5 | 56-67 | NRLNRLTTDFNVIVE |
| 6 | 257-269 | GEIKWIDFVRGAK |
| 7 | 325-344 | SLNKWKSKGRRFKGKGKGNK |
| 8 | 292-303 | GNLQLRNKEVTW |
| 9 | 154-162 | IFVVFDSIE |
| 10 | 176-190 | KETDLLILFKDDYFA |
| 11 | 104-120 | YKNDVKNRSVYIKGFPT |
| 12 | 63-71 | TDFNVIVEA |
| 13 | 270-280 | EGIILFKEKAK |
| 14 | 354-367 | KVQFQGKKTKFASD |
| 15 | 246-253 | REDLHILF |
| 16 | 232-239 | CLLKFSGD |
| 17 | 379-386 | TGPVKRAR |
| 18 | 200-209 | KVEAKLRAKQ |
| Position refers to amino acids in the La/SSB sequence as numbered from the N terminus. Epitopes are defined as having reactivity of at least 3.5 S.d. above background in at least three anti-La/SSB patient sera. Epitopes are numbered from greatest to least in order of the extend of binding. Octapeptides of each putative epitope with the greatest binding are underlined. In situations in which two octapeptides within a particular epitope have very similar binding, both are underlined. | | |

### Binding of Affinity Purified Anti-La/SSB Antibodies.

Anti-La/SSB antibodies from two patients have been affinity purified on La/SSB cross-linked immunosorbents. The affinity enriched anti-La/SSB preparation from patient E11 is specifically increased in anti-La/SSB binding activity relative to the serum from which it was prepared. The affinity enriched preparation binds 17 of the 18 previously identified epitopes, the exception being epitope 17 (octapeptide 379); however, this preparation does bind octapeptides adjacent to epitope 17, particularly octapeptides 380 and 382. Those putative epitopes in which the binding is most enriched include epitopes 2, 3, 6, 9, 10, 12, 13 and 15. These same epitopes appear enriched whether peak or average reactivities for each putative epitope are compared. Essentially the same results have been obtained from an affinity enriched preparation of the anti-La/SSB from a second patient. This demonstration of enhanced binding with increased anti-La/SSB specific activity is powerful evidence that at least these peptide sequences represent linear epitopes of the anti-La/SSB autoimmune response.

### Reproducibility of La/SSB Pin ELISA.

The reproducibility of anti-Ro/SSA reference serum binding to the Ro/SSA octapeptide (EYRKKMDI) positive control pins has been followed throughout the study, giving an average standard deviation between assays of 14.0% and 16.3% for pins incubated in 1:100 and 3:100 anti-Ro/SSA reference serum, respectively. Sera with and without anti-La/SSB antibodies have also given equally consistent results when repeated in the pin ELISA.

Shared Sequences with Cardiac related Proteins. Consecutive amino acid sequence identity is shared between the heavy chain of human cardiac β-myosin and three linear regions in the La/SSB sequence, two of which coincide with La/SSB epitopes. Within epitopes 13 (La/SSB amino acids 277 through 280), a four amino acid sequence is shared with cardiac β-myosin (sequence residues 453 through 456), while there is a shared pentapeptide within epitope 18 (204 through 208) and cardiac β-myosin (619 through 623). Three of five homologous sequences shared with the M6 protein of *Streptococcus pyogenes,* the bacterium associated with rheumatic heart disease, fall within the La/SSB epitopes. Specifically, two pentapeptide matches occur in La/SSB epitopes 5 (59 through 63) and 18 (203 through 207), while a tetrapeptide match is within epitope 13 (276 through 279). Strikingly, epitopes 13 and 18 also share amino acid sequence with the B1 chain of laminin, an adherent glycoprotein found in all basement membranes, including the sarcolemmal membrane of heart. The region within epitope 13 contains two overlapping tetrapeptide matches with laminin B1 (1202 through 1205 and 1367 through 1370), coinciding with La/SSB amino acid residues 277 through 280 and 275 through 278, respectively; while La/SSB epitope 18 (202 through 207) shares a six consecutive amino acids with laminin B1 (1467 through 1472). Antibodies binding to these epitopes were found in each of the seven sera from patients who had a child with CCHB.

### Example 2: Identification of Linear Epitopes of the 60 kD Ro/SSA Protein.

Peptides that represent linear epitopes for the 60 kD protein, including many originally presented in the earlier application, are shown in Table 2. These data represent the average result from seven normal sera, four anti-Ro/SSA precipitin positive sera, and the anti-Ro/SSA affinity purified autoantibody from the same four anti-Ro/SSA precipitin positive patient sera. The data has been multiplied by a constant in each case so that the magnitude of the binding could be compared at a constant IgG concentration, 100 micrograms IgG per milliliter in these results.

This study demonstrates that the anti-Ro/SSA peptide binding activity is enriched in parallel with the anti-Ro/SSA activity directed against the native molecule. The specific binding activity against both the antigenic peptides and the Ro/SSA antigen increased by about three-fold by the affinity enrichment procedure, indicating that peptide binding is part of the overall anti-Ro/SSA response.

The first and second columns of Table 2 identify the sequences bound above a threshold of A₄₁₀ of 0.3. This threshold was chosen because none of the peptides were bound by the normal sera by an average of greater than A₄₁₀ =0.3. The first column in Table 2 is from the average of four anti-Ro/SSA precipitin sera and the second is from the affinity enriched anti-Ro/SSA. In the third column are the antigenic sequences from which the antigenic octapeptides are composed. The superscript asterisk "*" identifies the octapeptide number of the octapeptide with the greatest binding of any contiguous collection all with A₄₁₀ of at least 0.3. This part of the sequence is also underlined. If two octapeptides have an A₄₁₀ within 1 % of each other then two octapeptide are identified with an asterisk and the amino acids contributing to both octapeptides are underlined.

**TABLE 2.**

| **Average binding of four anti-Ro/SSA sera and affinity enriched anti-Ro/SSA preparations to octapeptides constructed from the sequence of the 60 Kd Ro/SSA peptide greater than A**_{**410**} **of 0.3.** | | |
|---|---|---|
| **Anti-Ro/SSA Sera (Octapeptide Number)** | **Affinity Purified Anti-Ro/SSA (Octapeptide Number)** | **Sequence** |
| | 30* | MNRLHRFL |
| | 37-38* | LCFGSEGGT |
| 45* | 45*-48 | SEGGTYYIKEQ |
| 47-48* | | |
| | 76-78*-79 | EIKSFSQEGRT |
| 81-82 | 81-82* | SQEGRTTKQ |
| | 84*-85 | GRTTKQEPM |
| 106-108* | 105-108*-109 | ISTKQAAFKAVS |
| | 111*-112 | AFKAVSEVC |
| 126-130*-133 | 126-130*-133 | FTFIQFKKDLKESMK |
| 139*-140 | 138-139*-140* | SMKCGMWGRA |
| 143-145*-146 | 142-145*-146 | GMWGRALRKAIA |
| 165-169*-170 | 165-173*-180 | ALAVTKYKORNGWSHKDLLRLSH |
| 172-173* | | |
| 183-184* | 182-184*-185 | LLRLSHLKPSS |
| | 210* | HELYKEKA |
| 212* | 212*-213 | LYKEKALSV |
| 222* | 215-222* | KALSVETEKLLKYL |
| | 224* | KLLKYLEA |
| 231-234* | 229-234* | LEAVEKVKRTKDE |
| 257*-261 | 257*-263*-271 | HLLTNHLKSKEVWKALLQEMPL |
| 263-264*-265*-266 280*-283 | 280*-283 | ALLRNLGKMTA |
| | 285* | LGKMTANS |
| 308-313*-315*-316 | 308-313*-315*-317 | LCNEKLLKKARIHPFHI |
| 330-331*-339 | 330-331*-340 | TYKTGHGLRGKLKWRPDE |
| | 352* | ALDAAFYK |
| 355*-357 362*-365*-366 | 355*-367 | AAFYKTFKTVEPTGKRFLLA |
| | 379*-381 | ASMNORVLGS |
| | 398* | AMCMVVTR |
| | 414* | AFSDEMVP |
| | 420* | VPCPVTTD |
| | 433* | VLMAMSQI |
| | 445* | TDCSLPMI |
| 449*-450 | 447-449*-454 | CSLPMIWAQKTNTPA |
| 453* | | |
| | 472*-474 | TFAGGVHPAI |
| 482-484*-489 | 481-484*-489 | IALREYRKKMDIPAKL |
| 197-201*-203 | 197-198*-205 | NTKYITKGWKEVHEL |

### Example 3: Identification of Linear Epitopes of the 70 kD nuclear ribonucleoprotein.

The sequence of the 70 kD nuclear ribonucleoprotein (nRNP) is reported by R.A. Spritz, et al., in Nucleic Acids Res. 24: 10373-10391 (1987), the teachings of which are incorporated herein. The data analysis is done the same as for the Ro/SSA and La/SSB antigens. Each sequence identified represents the binding of antibody to octapeptides that exceed arbitrary criteria in the solid phase assay. The identified antigenic regions are more than two standard deviations above the mean of normal sera and are bound by more than half of the patients. For the binding to the 70 kD peptide, the threshold in the assay is A₄₁₀ of 0.380. The numbers in parentheses are the sequence positions of the first amino acid in the first octapeptide that exceeds the threshold. Each identified sequence is composed of one or more octapeptides. The number of octapeptides in each sequence is the length of the sequence minus eight. The underlined part of the sequence is the octapeptide that is bound most by the patient serum. In some cases, another octapeptide is essentially equivalent but not underlined.

The antigenic sequences of the 70 kD nRNP are: (11) ALFAPRDP, (65) ERMERKRREK, (133) HMVYSKRSGPRGY, (161) YVKHADGKKIDGRRVL, (178) VERGRTVK, (186) VKGWRPRR, (264) RRSRSRDK, (274) RRRSRERS, (277) SRERSKDK, (282) KDKDRDRKRRSSRSR, and (355) RRSHRSER.

The A peptide of nRNP has the following antigenic octapeptides: 916) NLNEKIKKD, (21) IKKDELKKSL, (44*) LVSRSLKMRGOAF, (73) QGFPFYDKPMRI, (93) IIAKMKGTF, (103*) ERDRKREKRKPKS, (116) QETPATKKA, (263) ALOGFKIT, and (274) AMKISFAKK. The threshold defined as two standard deviations above the mean is a normalized A₄₁₀ of 0.400. the octapeptide sequences identified by a (*) are bound by a subset of the sera tested. The sequences upon which this result is based is found in Sillekens, et al., EMBO J. 6:3841-3848 (1987), the teachings of which are incorporated herein.

The C peptide of nRNP has the following antigenic octapeptides: (19) SVRKTHCSGRKHKENVKD, (35) KDYYQKWM, (56) AFOOGKIPP, (61) KIPPTPFS, (78) PPPPSLPG, (82) SLPGPPRP, (85) GPPRPGMMPA, (108) PPPPGMMP, (117) GPAPGMRPP, (136) PPMMRPPA, and (152) PGMTRPDR. The threshold for binding of these octapeptides was A₄₁₀ of 0.440. The sequence upon which this result is based is in Sillekens, et al., Nucleic Acids Res. 25:8307-8321 (1988), the teachings of which are incorporated herein.

### Example 3(i): Linear Epitope Mapping of an Sm B/B' Polypeptide

Autoantibodies binding the Sm B/B' peptides are commonly associated with SLE IgG antibodies binding overlapping octapeptides of Sm B/B' have been evaluated in 10 patients with anti-Sm and anti-nRNP precipitins, 5 patients with other autoimmune serology, and 4 normal human sera. Neither normal controls nor patients without an anti-Sm precipitin significantly bind any of the Sm B/B' octapeptides. All sera tested containing an anti-Sm precipitin strongly bind octapeptides from eight regions of the Sm B/B' sequence. Three of these eight regions share the same octapeptide sequences (PPPGMRPP) that are consistently the most immunoreactive octapeptides from Sm B/B'. Binding of the similar PPPGIRGP, as well as binding to deletion and substitution peptides, suggest that the motif PPPG(I,M) (R,K) appears to best define this binding. PAPGMRPP in the nRNP C peptide is as antigenic as PPPGMRPP and may provide a partial explanation for the cross-reactivity shown between Sm and nRNP autoantibodies. However, the sequence PPPGMIPP from nRNP A is not antigenic. These data define the linear sequence autoantigenicity of the Sm B/B' protein. They also demonstrate that the predominant autoimmune epitope is a proline-rich sequence from which limited variance is permitted before antigenicity is destroyed.

Two sequences of Sm B/B' have been reported. Rokeach et al., J. Biol. Chem. 264:5024 (1989) have obtained a sequence from a lymphoblastoid cell (Raji) library that is identical to a partial clone from HeLa cells, reported by Sharpe, et al., FEB Lett. 250:585 (1989) and to Sm N from a human cerebellar library reported by Schmauss, et al., Nucleic Acids Res. 17:1733 (1989). Sm B and Sm B' have very similar amino acid sequences, van Dam, et al., EMBO J. 8:3853 (1989). Indeed, it appears, that Sm B and Sm B' are alternate splicing products from a common pre-mRNA.

In this study overlapping octapeptides of the encoding regions of both Sm B/B' sequences were synthesized by solid-phase peptide synthesis. Antigenicity of each octapeptide was determined with a variety of sera from patients with SLE and normal controls.

### Materials and Methods

*Sera.* Human sera form patients who satisfied the classification criteria of the American Rheumatism Association for SLE or normal agematched, sex-matched controls were used in this study. Fifteen sera from lupus patients were tested. Ten of these sera tested contained antibodies that formed strong precipitin lines with both Sm and nRNP, three formed precipitin lines with only nRNP, one formed a precipitin line with Ro/SSA and La/SSB, and one formed a precipitin line with only Ro/SSA. All of these serologic findings were confirmed by appropriate molecular weight bands for the reported antigen being bound in immunoblot by the respective sera.

*Solid-phase noncleavable peptide synthesis.* The published sequence of Sm B/B' was used to construct all the possible overlapping octapeptides. The amino acids used for peptide synthesis had Fmoc protected primary amino groups and t-butyl (or other appropriate group) protected side chains. Overlapping octapeptides were simultaneously synthesized at the rounded ends of radiation derivatized polyethylene pins that were arranged in the format of a 96 well microliter plate (Cambridge Research Biochemicals. Cambridge, UK and Coselco Mimotopes Pty Ltd. Victoria, Australia) The active esters of Fmoc, t-butyl amino acid solutions (30 mM) were solubilized in DMF that had 10 hydroxybenzotriazole added to a final concentration of 30 mM and dispensed into the wells of a microliter plate. Each amino acid was added as determined by the 240 amino acids of the Raji Sm B/B' sequence. After 18 h of incubation, the pins were washed in DMF for 5 min. four times at 2 min each in methanol, and once again in DMF for 5 min. The Fmoc protecting groups were then removed from the newly added amino acid by a 20% piperidine/ DMF bath for 30 min. These steps were repeated until all eight amino acids were added. After the final amino acid, the amino terminal groups of each peptide was acetylated by incubating the pins in a 5:2:1 (v/v/v) mixture of DMF:acetic anhydride: triethylamine for 90 min at room temperature. After this step the pins were again washed in DMF for 2 min, four times at 2 min each in methanol, and then air-dried for 10 min. Finally, side chain amino protecting groups were removed by 95:2, 5:2.5 (v/w/v) of trifluoracetic acid:phenol:ethanedithiol. Wash steps included 2 min in methylene chloride, two times at 5 min each in 5% diisopropylethylene/methylene chloride, and a final methylene chloride wash for 5 min. After drying for 10 min., pins were placed in distilled water for 2 min., transferred to a methanol bath for 18 h. and dried under vacuum for 18 h. This procedure was repeated to synthesize another set of the carboxyl-terminal 36 octapeptides to allow substantiation of the previous data. In addition other octapeptides, including the amino acid changes seen in the vanDam/ Ohosone Proc. Natl. Acad. Sci. USA 86:4249 (1989) sequences, were also synthesized. Selected substitution and deletion sequences, along with similar sequences from other proteins, were also constructed. Control pins composed of amino acids in a random sequence were prepared that were not present in any of the Sm or nRNP antigen sequences. In addition, positive control pins were synthesized from a known reactive sequence of the La/SSB peptide.

*Solid-phase cleavable peptide synthesis.* Coselco Mimotopes Pty Ltd. has developed a method of producing cleavable peptides. These peptides are synthesized on polyethylene pins that contain a cleavable linker assembly that contains an additional proline-lysine-alanine sequence. After synthesizing the peptide as described above, a final 15-min sonication in 0.1 % HCI (in 1:1 methanol/ddH₂0) is added. Pins are incubated in a solution of equal amounts of 0.1 M citrate and 0.1 M phosphate buffer at pH 3.0 for 3 h with gentle agitation. This removes all residual contaminants. Peptides are then cleaved from the pins by incubation in a 0.1 M phosphate solution at ph 7.0 that causes cyclization and formation of a diketopiperazole. These peptides were then analyzed for amino acid content. Four octapeptides were constructed as described and the correct amino acids in the proper ratios were present in all peptides tested.

*Solid phase antipeptide assay.* Wash steps and incubations were carried out in sealed plastic containers. Other assay steps were performed by lowering the pins into microliter plate wells. First, pins were blocked with 3 % low-fat milk in PBS for 1 h at room temperature. Pins were then incubated in 1/100 dilutions of sera in 3% milk/PBS with 0.05% Tween overnight at 4°C in humidified sealed containers. The pin blocks were then washed four times with PBS with 0.05% Tween for 10 min each with vigorous agitation. Next, each pin was incubated with anti-human gamma-chain specific IgG raised in a goat, affinity purified and conjugated to alkaline phosphatase (Jackson Immunoresearch Laboratories, West Grove, PA) at a 1/10,000 dilution. Paranitrophenyl phosphate disodium was used as a substrate for alkaline phosphatase and plates were read at 405 nm with a Microelisa Reader (Dynatech, Alexandria, VA). Results for each plate were then standardized by comparison with positive control pins. The same control pins were used for all plates and were allowed to develop to a specific OD with a known concentration of a standard control sera.

After completion of an assay, pins were sonicated for 2 h in sonication buffer (40 g SDS, 4 ml β-mercaptoethanol, and 62.4 g sodium phosphate to 4 liters) to remove antibodies, conjugate, and substrate. After sonication pins were washed twice in hot water and boiled in methanol for 2 min. Pins were then allowed to air dry for a minimum of 10 min and were stored with desiccant or used for another assay.

*Epitope mapping of Sm B/B'.* Peptides were first screened for reactivity with anti-human IgG conjugate alone. No background was demonstrated with anti-human IgG conjugate. Four normal human sera also showed minimal background reactivity with no specific antigenic regions demonstrated. SLE patients with autoimmune serology other than anti-Sm, anti-nRNP also showed no convincing, specific reactivity with any of the octapeptides. Other rheumatic serology tested included sera which formed precipitins with Ro/SSA as well as with both Ro/SSA and La/SSB Ag. Every patient who precipitated Sm and nRNP, however, showed considerable reactivity with various regions of the Sm B/B' protein. Ten Sm, nRNP sera were tested and all had a similar pattern of binding. Considerable reactivity was demonstrated in the proline-rich, carboxyl-terminal region of the protein. A repeated motif, PPPGMRPP, is found in three regions of the Sm B/B' polypeptide and is similarly antigenic in each. In addition, a closely related fourth region, PPPGIRGP, is bound by all the Sm, nRNP sera tested.

Several other antigenic regions were also detected in the first and middle portions of the polypeptide. These regions were not strongly reactive in all sera tested; however, they may be important by defining the differences in the very fine specificity between individuals with an autoimmune response to the B/B' Ag.

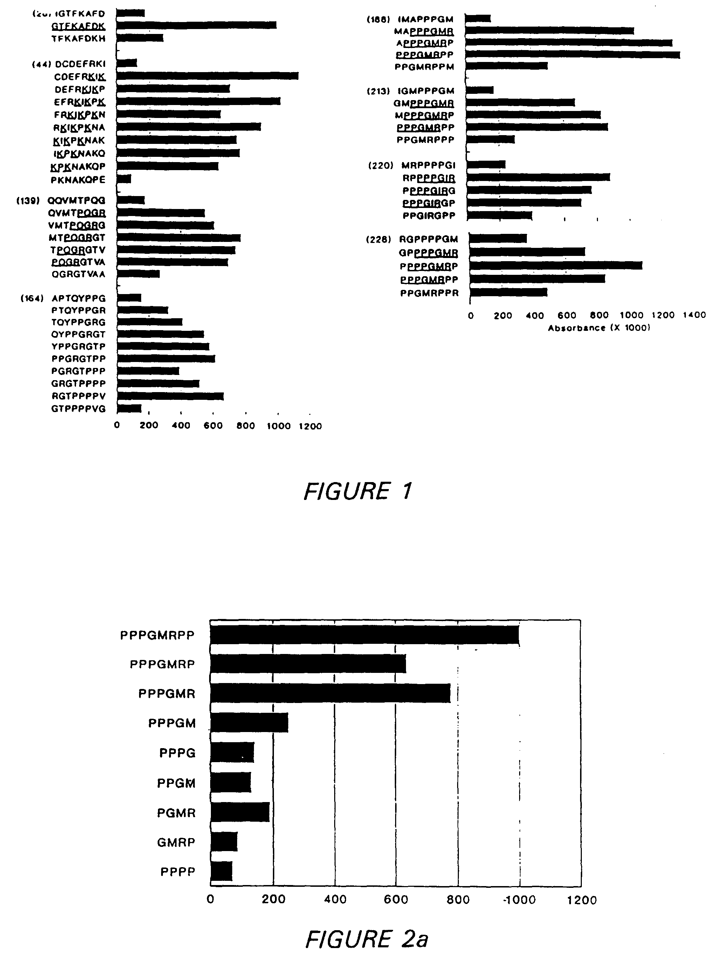

To elucidate which amino acids are essential for reactivity the average binding surrounding each purported epitope is presented along with the sequence of each relevant octapeptide in Figure 1. The octapeptide starting with amino acid 29, GTFKAFDK, requires all eight residues for reactivity. The antigenicity of the reactive area in the region of octapeptide 45, however, appears to be based on a requirement for two lysines with an intercalated amino acid spacer. Binding is lost in octapeptides 44 and 53 when the two lysines with intercalated spacer is eliminated.

The sequence from octapeptides 140 to 145 all have moderately elevated average binding. Each shares a PQGR sequence that would appear to be critical for binding. The reactive site surrounding octapeptide 169, on the other hand, is not easily explained by a specific sequence. The repeated PPPGMRPP and the similar PPPGIRGP found at octapeptides 191, 216, 223, and 231 share the hexamer, PPPGXR, where X indicates an undetermined amino acid, in all of the octapeptides with the greatest binding. This repeated motif appears to be important in the binding of anti-Sm to linear epitopes of Sm B/B'.

*Deletion experiments with PPPGMRPP.* Deletion experiments of the PPPGMRP sequence demonstrated that not all eight amino acids are required for antigenicity with the six patient sera tested, as shown in Figure 2a. Peptides were synthesized that deleted the carboxyl-terminal amino acid leaving a heptamer of PPPGMRP, a hexamer of PPPGMR, a pentamer of PPPGM, and various tetrameres including PPPG, PPGM, PGMR, GMRP, and PPPP. Binding appears to require at least the hexamer PPPGMR before the significant reactivity is lost. Greater than 60% of the reactivity to the PPPGMRPP motif is destroyed when the sixth position arginine is removed. Removal of the carboxyl terminal prolines does not appear to significantly alter binding in the six patients tested. In addition no solely poly-proline sequence tested (PPPPP, PPPP, PPP, PP) has shown reactivity with these sera. Six of the 10 sera containing anti-Sm and anti-nRNP were tested with these deleted peptides and gave similar results. A normal serum did not bind any of these peptides.

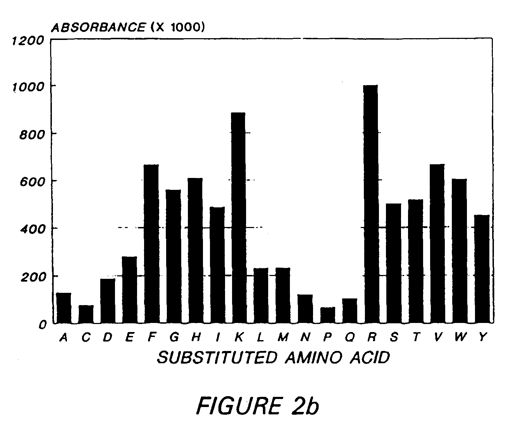

*Substitution experiments of PPPGMRPP antigenic sequence.* Substitution of the arginine (R) with the other 19 naturally occurring amino acids demonstrated varying levels of reactivity with the six sera tested, as shown in Figure 2b. Substitution of the arginine by another positively charged amino acid, lysine (K), preserves more than 75% of the original reactivity. Another subset of the nine amino acids (containing F, G, H, I, S, T, V, W, and Y) retain approximately 50 to 65% of original binding, whereas the other nine amino acids reduce binding to less than 25%.

*Synthesis of various similar epitopes.* Short sequences of various proteins that displayed considerable homology with the PPPGMRPP motif were also synthesized on polyethylene pins. These sequences and their reactivity, as measured by six different patient sera with anti-Sm and anti-nRNP precipitins from the first experiments. Octapeptides from nRNP A (23), nRNP C (24), and the EBV nuclear Ag-1 (25) were prepared. The nRNP A and one nRNP C sequence showed relatively no reactivity; however, the second nRNP C sequence (PAPGMRPP) showed over 90% of the original binding. In addition, the EBV sequence (which has five of six amino acid homology with the required antigenic portion of PPPGMRPP) is also significantly bound by Sm, nRNP precipitin positive patients.

All antigenic sites determined in this study require a positively charged amino acid. The size of these sites varies from two nonconsecutive basic amino acids to an entire octapeptide. The PPPGMRPP motif appears to be the major linear antigenic epitope in all Sm, nRNP sera tested; however, this sequence does not appear to be bound by sera that contain an anti-Sm precipitin in the absence of an anti-nRNP precipitin. Sm precipitin alone patients bind two regions of Sm B/B'.

The carboxyl-terminal di-proline of the PPPGMRPP motif is not required for antigenicity. Other deletion studies have also shown that the antigenicity of these autoantibodies are not specifically directed against the poly-proline regions. Peptides containing from two to six polyprolines have shown no reactivity with the sera used in this study. In addition, naturally occurring octapeptides of amino acid sequences 175-186 and 85-92 contain three or four consecutive prolines: none of these regions is antigenic. Also the very similar PPPGMIPP octapeptides does not bind to the sera precipitating Sm and nRNP.

Substitution experiments have shown that changing one amino acid can reduce reactivity of a sequence by more than 90%. Substituting the other 19 naturally occurring amino acids for the sixth amino acid, arginine (R), of the PPPGMRPP sequence has divided these amino acids into three separate groups. Lysine (K), another basic amino acid, is the only substitution that preserves an average of more than 75% of the binding. With each of the sera tested, this amino acid substitution is consistently the most reactive. However, reactivity is ablated to less than 25 % with nine amino acids. This set includes the acidic amino acids and their amines, the sulfur-containing amino acids, and the hydrophobic leucine, proline, and alanine. The other nine amino acids retain part, 42 to 65%, of the original binding. Phenylalanine was consistently the most reactive of this group. Nevertheless, the ability of lysine to substitute for arginine leads to the hypothesis that a positively charged amino acid at this position is preferred for the antigenicity of this sequence.

### Example 4: Binding of monoclonal antibodies against peptide determinants of Sm B/B'.

Autoantibodies binding the Sm B and B' peptides (B/B') are commonly associated with systemic lupus erythematosus in man and in MRL *lpr/lpr* mice. KSm 3 and KSm 5 were derived from an unmanipulated MRL *lpr/lpr* mouse using hybridoma monoclonal antibody technology. Supernatants containing KSm 3 and KSm 5 monoclonal antibodies were collected from cloned murine hybridoma cell lines in RPMI 1640 with 10% fetal bovine serum, glutamine, penicillin and streptomycin. These monoclonal autoantibodies are both of the IgG2a subclass. The linear antigenic regions of these two anti-Sm B/B' murine monoclonal autoantibodies have been mapped using overlapping octapeptides. Unique epitopes are identified by each monoclonal. Monoclonal antibody KSm 5 recognizes the peptide, PPPGMRPP, which is repeated three times in the Sm B polypeptide. KSm 3 binds best to two very similar, almost neighboring octapeptides, PPPGIRGP and PGIRGPPP. The two monoclonals do not crossreact. Both of these regions of Sm B/B' are major areas of antigenicity for human anti-Sm autoantibodies. Amino acid deletion and substitution in antigenic octapeptides show that binding to the KSm 5 epitope is lost with only slight modification. When the arginine in the sixth position PPPGMRPP is substituted KSm 5 binding is found in an unexpected subset of octapeptides. Molecular dynamic modelling suggests that binding may be associated with a shared peptide backbone structure rather than charge or hydrophobicity of the substituted amino acid. In contrast, binding of KSm 3 to PPPGIRPP is abolished when the sixth position arginine is substituted by any other amino acid. These two murine autoantibodies bind distinct linear epitopes of Sm B/B' which are also bound by human anti-Sm B/B' autoantibodies. The bound epitopes are proline rich and contain an arginine in the fourth or sixth position. Thus, substitution at arginine and modelling experiments suggest very different mechanisms of binding for KSm 3 and KSm 5. A particular peptide backbone conformation, conferred by some amino acid sidechains at Position 6 of the octapeptide PPPGMRPP, may be involved in KSm 5 binding while KSm 3 binding requires the specificity of the arginine side chain. Naturally arising autoantibodies may bind quite different features of similar peptide structures.

Substitution of either the fifth or sixth position amino acids of PPPGIRGP with all of the other nineteen naturally occurring amino acids shows unique patterns of reactivity. Substitution of the sixth amino acid arginine (R) shows that no other amino acid allows any degree of binding. When the fifth position isoleucine (I) is substituted, six of the twenty amino acids, phenylalanine (F), histidine (E) threonine (T), valine (V) and tyrosine (Y), allow over 50% of the binding shown with the original octapeptide. Of these, threonine has more reactivity than the original octapeptide.

To determine structural motif common to peptides of either antigenic group, molecular dynamic simulations were performed for selected peptides which are antigenic to KSm 5 or KSm 3. A total of 2500 structures representing a nanosecond trajectory in time were accumulated for each peptide, and analyzed by monitoring backbone dihedral angles. At least one prevalent conformer could be chosen for all peptides as observed by a stable set of backbone dihedral angles. The root mean square difference between the KSm 3 positive peptides PPPGIRGP and PGIRGPPP of 4.87 A for the backbone atoms of eight residues indicates that there is no common backbone between these two peptides.

In contrast, the binding of KSm 5 to peptides substituted for arginine (R) in the sixth position in PPPGMRPP suggest quite different binding requirements. Amino acids with side chains, -(CH₃)₃-NH-C-NHNH₂, -CH₃, -OH, and -H, all were roughly-equivalent in their ability to be bound by KSm 5. No feature of charge or hydrophobicity is shared by these amino acids. Also, lysine and histidine, the amino acids usually considered most similar to arginine, could not substitute and preserve binding. The possibility of a significant backbone conformation common to the native peptide PPPGMRPP off set by one or more amino acids in either direction was ruled out using OVRLAP18. The best match was found for the first few residues of the backbone with no offset. Accordingly, the conformation may play a role in binding.

## Claims

1. A peptide forming a linear epitope for a human autoantibody selected from either (i) the group of peptides of less than forty amino acids, wherein the sequence of the epitope begins with the amino acid numbered from the amino terminus followed by the listed amino acid sequence consisting of
the La/SSB epitopes:
ICHQIEYYFGDFNLPRDKFLK (Sequence Listing ID No. 20),
YFGDFNLP (amino acids 8-15 of Sequence Listing ID No. 20),
WVPLEIMIKFNR (Sequence Listing ID No. 21),
VPLEIMIK (amino acids 2-9 of Sequence Listing ID No. 21),
NRLNRLTTDFNVIVE,(Sequence Listing ID No. 23),
NRLNRLTT (amino acids 1-8 of Sequence Listing ID No. 23),
TDFNVIVE (Sequence Listing ID No. 30),
DFNVIVEA (amino acids 2-9 of Sequence Listing ID No. 30),
KTKIRRSPSKPL (Sequence Listing ID No. 22),
KIRRSPSK (amino acids 4-11 of Sequence Listing ID No. 22),
YKNDVKNRSVYIKGFPT (Sequence Listing ID No. 29),
SVYIKGFP (amino acids 9-16 of Sequence Listing ID No. 29),
QVLNIQMRRTLHKAFKGS (Sequence Listing ID No. 19),
NIQMRRTLHKAFK (amino acids 4-16 of Sequence Listing ID No. 19),
RTLHKAFK (amino acids 9-16 of Sequence Listing ID No. 19),
IFVVFDSIE (Sequence Listing ID No. 27),
FVVFDSIE (amino acids 2-9 of Sequence Listing ID No. 27),
KETDLLILFKDDYFA (Sequence Listing ID No. 28),
ILFKDDYF (amino acids 7-14 of Sequence Listing ID No. 28),
KVEAKLRAKQ (Sequence Listing ID No. 36),
EAKLRAKQ (amino acids 3-10 of Sequence Listing ID No. 36);
CLLKFSGD (Sequence Listing ID No. 34),
REDLHILF (Sequence Listing ID No. 33),
GEIKWIDFVRGAK (Sequence Listing ID No. 24),
KWIDFVRGAK (amino acids 4-13 of Sequence Listing ID No. 24),
IDFVRGAK (amino acids 6-13 of Sequence Listing ID No. 24),
EGIILFKEKAK (Sequence Listing ID No. 31),
EGIILFKE (amino acids 1-8 of Sequence Listing ID No. 31),
GNLQLRNKEVTW (Sequence Listing ID No. 26),
LRNKEVTW (amino acids 5-12 of Sequence Listing ID No. 26),
SLNKWKSKGRRFKGKGKGNK (Sequence Listing ID No. 25),
KSKGRRFK (amino acids 6-13 of Sequence Listing ID No. 25),
KVQFQGKKTKFASD, (Sequence Listing ID No. 32),
KKTKFASD (amino acids 7-14 of Sequence Listing ID No. 32),
TGPVKRAR (Sequence Listing ID No. 35),
the Ro/SSA epitopes:
MNRLHRFL (Sequence Listing ID No. 37),
LCFGSEGGT (Sequence Listing ID No. 38),
CFGSEGGT (amino acids 2-9 of Sequence Listing ID No. 38),
SEGGTYYIKEQ (Sequence Listing ID No. 39),
EGGTYYIKEQ (amino acids 2-11 of Sequence Listing ID No. 39),
GTYYIKEQ (amino acids 4-11 of Sequence Listing ID No. 39),
GTYYI (amino acids 4-8 of Sequence Listing ID No. 39),
EIKSFSQEGRT (Sequence Listing ID No. 40),
KSFSQEGR (amino acids 3-10 of Sequence Listing ID No. 40),
SQEGRTTKQ (Sequence Listing ID No. 41),
GRTTKQEPM (Sequence Listing ID No. 42),
STKQAAFKAV (amino acids 2-11 of Sequence Listing ID No. 43),
ISTKQAAFKAVS (Sequence Listing ID No. 43),
KQAAFKAV (amino acids 4-11 of Sequence Listing ID No. 43),
AFKAVSEVC (Sequence Listing ID No. 44),
FTFIQFKKDLKESMK (Sequence Listing ID No. 45),
QFKKDLKE (amino acids 5-12 of Sequence Listing ID No. 45),
SMKCGMWGRA (Sequence Listing ID No. 46),
MKCGMWGRA (amino acids 2-10 of Sequence Listing ID No. 46),
GMWGRALRKAIA (Sequence Listing ID No. 47),
GRALRKAI (amino acids 4-11 of Sequence Listing ID No. 47),
ALAVTKYKQRNGWSHKDLLRLSH (Sequence Listing ID No. 48),
TKYKQRNG (amino acids 5-12 of Sequence Listing ID No. 48),
QRNGWSHK (amino acids 9-16 of Sequence Listing ID No. 48),
LLRLSHLKPSS (Sequence Listing ID No. 49),
RLSHLKPS (amino acids 3-10 of Sequence Listing ID No. 49),
TKYITKGW (amino acids 2-9 of Sequence Listing ID No. 71),
ITKGWKEV (amino acids 5-12 of Sequence Listing ID No. 71),
HELYKEKA (Sequence Listing ID No. 50),
LYKEKALSV (Sequence Listing ID No. 51),
KALSVETEKLLKYL (Sequence Listing ID No. 52),
TEKLLKYL (amino acids 7-14 of Sequence Listing ID No. 52),
KLLKYLEA (Sequence Listing ID No. 53),
LEAVEKVKRTKDE (Sequence Listing ID No. 54),
KVKRTKDE (amino acids 6-13 of Sequence Listing ID No. 54),
HLLTNHLKSKEVWKALLQEMPL (Sequence Listing ID No. 55),
LKSKEVWK (amino acids 7-14 of Sequence Listing ID No. 55),
KSKEVWKA (amino acids 8-15 of Sequence Listing ID No. 55),
SKEVWK (amino acids 9-14 of Sequence Listing ID No. 55),
ALLRNLGKMTA (Sequence Listing ID No. 56),
RNLGKMT (amino acids 4-10 of Sequence Listing ID No. 56),
LGKMTANS (Sequence Listing ID No. 57),
LCNEKLLKKARIHPFHI (Sequence Listing ID No. 58),
LLKKARI (amino acids 6-12 of Sequence Listing ID No. 58),
KKARIHPF (amino acids 8-15 of Sequence Listing ID No. 58),
TYKTGHGLRGKLKWRPDE (Sequence Listing ID No. 59),
YKTGHGL (amino acids 2-8 of Sequence Listing ID No. 59),
ALDAAFYK (Sequence Listing ID No. 60),
AAFYKTFKTVEPTGKRFLLA (Sequence Listing ID No. 61),
ASMNQRVLGS (Sequence Listing ID No. 62),
EPTGKRFL (amino acids 11-18 of Sequence Listing ID No. 61),
AMCMVVTR (Sequence Listing ID No. 63),
AFSDEMVP (Sequence Listing ID No. 64),
VPCPVTTD (Sequence Listing ID No. 65),
VLMAMSQI (Sequence Listing ID No. 66),
TDCSLPMI (Sequence Listing ID No. 67),
LPMIWAQKTNTPA (amino acids 3-15 of Sequence Listing ID No. 68),
TFAGGVHPAI (Sequence Listing ID No. 69),
TFAGGVHP (amino acids 1-8 of Sequence Listing ID No. 69),
IALREYRKKMDIPAKL (Sequence Listing ID No. 70),
REYRKKMD (amino acids 4-11 of Sequence Listing ID No. 70);
the 70 kD nRNP epitopes:
ALFAPRDP (Sequence Listing ID No. 72),
ERMERKRR (Sequence Listing ID No. 73),
MVYSKRSG (Sequence Listing ID No. 74),
GKKIDGRR (Sequence Listing ID No. 75),
VERGRTVK (Sequence Listing ID No. 76),
VKGWRPRR (Sequence Listing ID No. 77),
RRSRSRDK (Sequence Listing ID No. 78),
RRRSRERS (Sequence Listing ID No. 79),
SRERSKDK (Sequence Listing ID No. 80),
KRRSSRSR (Sequence Listing ID No. 81), and
RRSHRSER (Sequence Listing ID No. 82);
the nRNP A peptide epitopes:
LNEKIKKD (Sequence Listing ID No. 83),
KKDELKKS (Sequence Listing ID No. 84),
SRSLKMRG (Sequence Listing ID No. 85),
PFYDKPMR (Sequence Listing ID No. 86),
IIAKMKGTF (Sequence Listing ID No. 87),
DRKREKRK (Sequence Listing ID No. 88),
QETPATKK (Sequence Listing ID No. 89),
ALQGFKIT (Sequence Listing ID No. 90), and
MKISFAKK (Sequence Listing ID No. 91); and
the nRNP C peptide epitopes:
RKHKENVK (Sequence Listing ID No. 92),
KDYYQKWN (Sequence Listing ID No. 93),
AFQQGKIP (Sequence Listing ID No. 94),
KIPPTPFS (Sequence Listing ID No. 95),
PPPPSLPG (Sequence Listing ID No. 96),
SLPGPPRP (Sequence Listing ID No. 97),
PPRPGMMP (Sequence Listing ID No. 98),
PPPPGMMP (Sequence Listing ID No. 99),
GPAPGMRP (Sequence Listing ID No. 100),
PPMMRPPA (Sequence Listing ID No. 101), and
PGMTRPDR (Sequence Listing ID No. 102);
or (ii) a group of peptides as defined in (i) consisting of between four and twenty five amino acids, but not including peptides having an amino acid sequence consisting of A I A L R E Y R K K M D I P A, V H P A I A L R, K L G L E N, K D L K, K S K E, L T A L L R, R G K L K W, L K A L D, T R T E K D S, D E M V, E Y R K K M D, A A A M, D P D D, L S H L K, R T K D E, E K D S, E V C R, nor peptides corresponding to residues 1-11, 35-58, 56-77, 74,96, 131-153, 149-166, 163-184, 180-205, 203-225 and 257-282 in the sequence of sn RNP UIA; and wherein peptides consisting of four to seven amino acids are contained within the amino acid sequences defined in (i) as the beginning of the epitope.

2. A peptide from group (i) as claimed in Claim 1.

3. A peptide from group (ii) as claimed in Claim 1 consisting of from six to twenty five amino acids.

4. Peptides of claim 1 reactive with anti-Ro/SSA polyclonal antibodies or anti-La/SSB polyclonal antibodies.

5. Peptides of claim 1 in a pharmaceutically acceptable carrier and an effective amount to elicit an immunogenic effect in a human or block autoantibodies wherein the peptide is selected from the group consisting of
GTFKAFDK (Sequence Listing ID No. 1),
TFKAFDKHM (Sequence Listing ID No. 15),
CDEFRKfIUKNAKQP (Sequence Listing ID No. 2),
EGPPPKDT (Sequence Listing ID No. 16),
KDTGIARV,(Sequence Listing ID No. 17), and
RVPLAGAA (Sequence Listing ID No. 3),
AGGPGVGRAAGRGVPAG (Sequence Listing ID No. 4),
IPQAPAGLAG (Sequence Listing ID No. 18),
AGLAGPVRGVGGPSQ (Sequence Listing ID No. 5),
QVMTPQGRGTVA (Sequence Listing ID No. 6),
PTQYPPGRGTPPPPV (Sequence Listing ID No. 7),
TPPPPVGRATPPPGI (Sequence Listing ID No. 8),
PPPGIMAP (Sequence Listing ID No. 9),
MAPPPGMRPPM (Sequence Listing ID No. 10),
PIGLPPARGTPIGMPP (Sequence Listing ID No. 11),
PIGMPPPG (Sequence Listing ID No. 12),
RPPPPGIRGPP (Sequence Listing ID No. 13),
RGPPPPGMRPPR (Sequence Listing ID No. 14), or mixtures thereof.

6. Peptides of any of claims 1-4 in combination with a pharmaceutical carrier for administration to a patient.

7. Peptides of claim 6 in an effective concentration for administration to a patient to neutralize circulating autoantibody.

8. Peptides of claim 6 or 7 further comprising a pharmaceutical carrier for administration to a patient, wherein the carrier and concentration of sequences elicit an immune response when administered to a host.

9. Peptides of any of claims 1 to 5 labelled with a compound selected from the group consisting of dyes, fluorescent labels, chemiluminescent labels, enzymes, and radioactive labels.

10. Peptides of any of claims 1 to 5 immobilized onto a substrate.

11. An ex vivo method for screening patients for autoimmune disorders comprising reacting a biological sample taken from a patient with a peptide as claim in any of claims 1-5.

12. The method of claim 11 further comprising detecting autoantibodies in the patient sample.

13. The method of claim 12 further comprising predicting the prognosis of the patient based on the reactivity of the patient sample with the peptides.

14. A peptide as claimed in any of claims 1 to 10 for use in medicine.

15. Use of a peptide as claimed in any of claims 1 to 10 in the manufacture of a medicament for use in a method for treating patients for autoimmune disorders comprising administering the medicament to the patient.

16. The use of claim 15 wherein the peptide acts as a vaccine against an autoimmune disorder.

17. The use of claim 15 wherein the peptide binds to the autoantibodies to block the autoimmune response.

18. The use of claim 15 wherein the peptides are administered in combination to block an autoimmune response involving more than one autoantibody.

## Patentansprüche

1. Peptid, das ein lineares Epitop für einen humanen Autoantikörper bildet und das entweder (i) aus der Gruppe der Peptide mit weniger als 40 Aminosäuren, wobei die Sequenz des Epitops mit der vom Aminoterminus her numerierten Aminosäure beginnt, gefolgt von der angegebenen Aminosäuresequenz, bestehend aus
den folgenden La/SSB-Epitopen:
ICHQIEYYFGDFNLPRDKFLK (Sequenzprotokoll ID No. 20),
YFGDFNLP (Aminosäuren 8-15 des Sequenzprotokolls ID No. 20),
WVPLEIMIKFNR (Sequenzprotokoll ID No. 21),
VPLEIMIK (Aminosäuren 2-9 des Sequenzprotokolls ID No. 21),
NRLNRLTTDFNVIVE (Sequenzprotokoll ID No. 23),
NRLNRLTT (Aminosäuren 1-8 des Sequenzprotokolls ID No. 23),
TDFNVIVE (Sequenzprotokoll ID No. 30),
DFNVIVEA (Aminosäuren 2-9 des Sequenzorotokolls ID No. 30),
KTKIRRSPSKPL (Sequenzprotokoll ID No. 22),
KIRRSPSK (Aminosäuren 4-11 des Sequenzprotokolls ID No. 22),
YKNDVKNRSVYIKGFPT (Sequenzprotokoll ID No. 29),
SVYIKGFP (Aminosäuren 9-16 des Sequenzprotokolls ID No. 29),
QVLNIQMRRTLHKAFKGS (Sequenzprotokoll ID No. 19),
NIQMRRTLHKAFK (Aminosäuren 4-16 des Sequenzprotokolls ID No. 19),
RTLHKAFK (Aminosäuren 9-16 des Sequenzprotokolls ID No. 19),
IFVVFDSIE (Sequenzprotokoll ID No. 27),
FVVFDSIE (Aminosäuren 2-9 des Sequenzprotokolls ID No. 27),
KETDLLILFKDDYFA (Sequenzprotokoll ID No. 28),
ILPKDDYF (Aminosäuren 7-14 des Sequenzprotokolls ID No. 28),
KVEAKLRAKQ (Sequenzprotokoll ID No. 36),
EAKLRAKQ (Aminosäuren 3-10 des Sequenzprotokolls ID No. 36),
CLLKFSGD (Sequenzprotokoll ID No. 34),
REDLHILF (Sequenzprotokoll ID No. 33),
GEIKWIDFVRGAK (Sequenzprotokoll ID No. 24),
KWIDFVRGAK (Aminosäuren 4-13 des Sequenzprotokolls ID No. 24),
IDFVRGAK (Aminosäuren 6-13 des Sequenzprotokolls ID No. 24),
EGIILFKEKAK (Sequenzprotokoll ID No. 31),
EGIILFKE (Aminosäuren 1-8 des Sequenzprotokolls ID No. 31),
GNLQLRNKEVTW (Sequenzprotokoll ID No. 26),
LRNKEVTW (Aminosäuren 5-12 des Sequenzprotokolls ID No. 26),
SLNKWKSKGRRFKGKGNK (Sequenzprotokoll ID No. 25),
KSKGRRFK (Aminosäuren 6-13 des Sequenzprotokolls ID No. 25),
KVQFQGKKTKFASD (Sequenzprotokoll ID No. 32),
KKTKFASD (Aminosäuren 7-14 des Sequenzprotokolls ID No. 32),
TGPVKRAR (Sequenzprotokoll ID No. 35),
den folgenden Ro/SSA-Epitopen:
MNRLHRFL (Sequenzprotokoll ID No. 37),
LCFGSEGGT (Sequenzprotokoll ID No. 38),
CFGSEGGT (Aminosäuren 2-9 des Sequenzprotokolls ID No. 38),
SEGGTYYIKEQ (Sequenzprotokoll ID NO. 39),
EGGTYYIKEQ (Aminosäuren 2-11 des Sequenzprotokolls ID No. 39),
GTYYIKEQ (Aminosäuren 4-11 des Sequenzprotokolls ID No. 39),
GTYYI (Aminosäuren 4-8 des Sequenzprotokolls ID No. 39),
EIKSFSQEGRT (Sequenzprotokoll ID No. 40),
KSFSQEGR (Aminosäuren 3-10 des Sequenzprotokolls ID No. 40),
SQEGRTTKQ (Sequenzprotokoll ID No. 41),
GRTTKQEPM (Sequenzprotokoll ID No. 42),
STKQAAFKAV (Aminosäuren 2-11 des Sequenzprotokolls ID No. 43),
ISTKQAAFKAVS (Sequenzprotokoll ID No. 43),
KQAAFKAV (Aminosäuren 4-11 des Sequenzprotokolls ID No. 43),
AFKAVSEVC (Sequenzprotokoll ID No. 44),
FTFIQFKKDLXESMK (Sequenzprotokoll ID No. 45),
QFKKDLKE (Aminosäuren 5-12 des Sequenzprotokolls ID No. 45),
SMKCGMWGRA (Sequenzprotokoll ID No. 46),
MKCGMWGRA (Aminosäuren 2-10 des Sequenzprotokolls ID No. 46),
GMWGRALRKAIA (Sequenzprotokoll ID No. 47),
GRALRKAI (Aminosäuren 4-11 des Sequenzprotokolls ID NO. 47),
ALAVTKYKQRNGWSHKDLLRLSH (Sequenzprotokoll ID No. 48),
TKYKQRNG (Aminosäuren 5-12 des Sequenzprotokolls ID No. 48),
QRNGWSHK (Aminosäuren 9-16 des Sequanzprotokolls ID No. 48),
LLRLSHLKPSS (Sequenzprotokoll ID No. 49),
RLSHLKPS (Aminosäuren 3-10 des Sequenzprotokolls ID No. 49),
TKYITKGW (Aminosäuren 2-9 des Sequenzprotokolls ID No. 71),
ITKGWKEV (Aminosäuren 5-12 des Sequenzprotokolls ID No. 71),
HELYKEKA (sequenzprotokoll ID No. 50),
LYKEKALSV (Sequenzprotokoll ID No. 51),
KALSVETEKLLKYL (Sequenzprotokoll ID No. 52),
TEKLLKYL (Aminosäuren 7-14 des Sequenzprotokolls ID No. 52),
KLLKYLEA (Sequenzprotokoll ID No. 53),
LEAVEKVKRTKDE (Sequenzprotokoll ID No. 54),
KVKRTKDE (Aminosäuren 6-13 des Sequenzprotokolls ID No. 54),
HLLTNHLKSKEVWKALLQEMPL (Sequenzprotokoll ID No. 55),
LKSKEVWK (Aminosäuren 7-14 des Sequenzprotokolls ID No. 55),
KSKEVWKA (Aminosäuren 8-15 des Sequenzprotokolls ID No. 55),
SKEVWK (Aminosäuren 9-14 des Sequenzprotokolls ID No. 55),
ALLRNLGKMTA (Sequenzprotokoll ID No. 56),
RNLGKMT (Aminosäuren 4-10 des Sequenzprotokolls ID No. 56),
LGKMTANS (Sequenzprotokoll ID No. 57),
LCNEKLLKKARIHPFHI (Sequenzprotokoll ID No. 58),
LLKKARI (Aminosäuren 6-12 des Sequenzprotokolls ID No. 58),
KKARIHPF (Aminosäuren B-15 des Sequenzprotokolls ID No. 58),
TYKTGHGLRGKLKWRPDE (Sequenzprotokoll ID No. 59),
YKTGHGL (Aminosäuren 2-8 des Sequenzprotokolls ID No. 59),
ALDAAFYK (Sequenzprotokoll ID No. 60),
AAFYKTFKTVEPTGKRFLLA (Sequenzprotokoll ID No. 61),
ASMNQRVLGS (Sequenzprotokoll ID No. 62),
EPTGKRFL (Aminosäuren 11-18 des Sequenzprotokolls ID No. 61),
AMCMVVTR (Sequenzprotokoll ID No. 63),
AFSDEMVP (Sequenzprotokoll ID No. 64),
VPCPVTTD (Sequenzprotokoll ID No. 65),
VLMAMSQI (Sequenzprotokoll ID No. 66),
TDCSLPMI (Sequenzprotokoll ID No. 67),
LPMIWAQKTNTPA (Aminosäuren 3-15 des Sequenzprotokolls ID No. 68),
TFAGGVHPAI (Sequenzprotokoll ID No. 69),
TFAGGVHP (Aminosauren 1-8 des Sequenzprotokolls ID No. 69),
IALREYRKKMDIPAKL (Sequenzprotokoll ID No. 70),
REYRKKMD (Aminosäuren 4-11 des Sequenzprotokolls ID No. 70),
die folgenden 70 kD-nRNP-Epitopen:
ALFAPRDP (Sequenzprotokoll ID No. 72),
ERMERKRR (Sequenzprotokoll ID No. 73),
MVYSKRSG (Sequenzprotokoll ID No. 74),
GKKIDGRR (Sequenzprotokoll ID No. 75),
VERGRTVK (Sequenzprotokoll ID No. 76),
VKGWRPRR (Sequenzprotokoll ID No. 77),
RRSRSRDK (Sequenzprotokoll ID No. 78),
RRRSRERS (Sequenzprotokoll ID No. 79),
SRERSKDK (Sequenzprotokoll ID No. 80),
KRRSSRSR (Sequenzprotokoll ID No. 81) und
RRSHRSER (Sequenzprotokoll ID No. 82),
den folgenden nRNP-A-Peptid-Epitopen:
LNEKIKKD (Sequenzprotokoll ID No. 83),
KKDELKKS (Sequenzprotokoll ID No. 84),
SRSLKMRG (Sequenzprotokoll ID No. 85),
PFYDKPMR (Sequenzprotokoll ID No. 86),
IIAKMKGTF (Sequenzprotokoll ID No. 87),
DRKREKRK (Sequenzprotokoll ID No. 88),
QETPATKK (Sequenzprotokoll ID No. 89),
ALQGFKIT (Sequenzprotokoll ID No. 90) und
MKISFAKK (Sequenzprotokoll ID No. 91), und
den folgenden nRNP-C-Peptid-Epitopen:
RKHENVK (sequenzprotokoll ID No. 92),
KDYYQKWN (Sequenzprotokoll ID No. 93),
AFQQGKIP (Sequenzprotokoll ID No. 94),
KIPPTPFS (Sequenzprotokoll ID No. 95),
PPPPSLPG (Sequenzprotokoll ID No. 96),
SLPGPPRP (Sequenzprotokoll ID No. 97),
PPRPGMMP (Sequenzprotokoll ID No. 98),
PPPPGMMP (Sequenzprotokoll ID No. 99),
GPAPGMRP (Sequenzprotokoll ID No. 100),
PPMMRPPA (Sequenzprotokoll ID No. 101) und
PGMTRPDR (Sequenzprotokoll ID No. 102);
oder (ii) aus der Gruppe von Peptiden gemäß Definition bei (i), die aus 4 bis 25 Aminosäuren bestehen, jedoch nicht Peptide mit einer aus A I A L R E Y R K K M D I P A, V H P A I A L R, K L G L E N, K D L K, K S K E, L T A L L R, R G K L K W, L K A L D, T R T E K D S, D E M V, E Y R K K M D, A A A M, D P D D, L S H L K, R T K D E, E K D S oder E V C R bestehenden Aminosäuresequenz noch Peptide, die den Resten 1-11, 35-58, 56-77, 74, 96, 131-153, 149-166, 163-184, 180-205, 203-225 oder 257-282 in der Sequenz von sn-RNP-UIA entsprechen, umfassen, wobei die Peptide aus 4-7 Aminosäuren in den bei (i) definierten Aminosäuresequenzen als Beginn des Epitops enthalten sind, ausgewählt ist.

2. Peptid aus der Gruppe (i) gemäß Anspruch 1.

3. Peptid aus der Gruppe (ii) gemäß Anspruch 1, das aus 6-25 Aminosäuren besteht.

4. Peptide nach Anspruch 1, die gegenüber polyklonalen anti-Ra/SSA-Antikörpern oder polyklonalen anti-La/SSB-Antikörpern reaktiv sind.

5. Peptide nach Anspruch 1 in einem pharmazeutisch akzeptablen Träger und in einer wirksamen Menge, um einen immunogenen Effekt bei einem Menschen hervorzurufen oder um Autoantikörper zu blockieren, wobei das Peptid aus
GTFKAFDK (Sequenzprotokoll ID No. 1),
TFKAFDKHM (Sequenzprotokoll ID No. 15),
CDEFRKIKPKNAKQP (Sequenzprotokoll ID No. 2),
EGPPPKDT (Sequenzprotokoll ID No. 16),
KDTGIARV (Sequenzprotokoll ID No. 17) und
RVPLAGAA (Sequenzprotokoll ID No. 3),
AGGPGVGRAAGRGVPAG (Sequenzprotokoll ID No. 4),
IPQAPAGLAG (Sequenzprotokoll ID No. 18),
AGLAGPVRGVGGPSQ (Sequenzprotokoll ID No. 5),
QVMTPQGRGTVA (Sequenzprotokoll ID No. 6),
PTQYPPGRGTPPPPV (Sequenzprotokoll ID No. 7),
TPPPPVGRATPPPGI (Sequenzprotokoll ID No. 8),
PPPGIMAP (Sequenzprotokoll ID No. 9),
MAPPPGMRPPM (Sequenzprotokoll ID No. 10),
PIGLPPARGTPIGMPP (Sequenzprotokoll ID No. 11)
PIGMPPPG (Sequenzprotokoll ID No. 12),
RPPPPGIRGPP (Sequenzprotokoll ID No. 13),
RGPPPPGMRPPR (Sequenzprotokoll ID No. 14)
oder Gemischen hiervon ausgewählt ist.

6. Peptide nach einem der Ansprüche 1-4 in Kombination mit einem pharmazeutischen Träger zur Verabreichung an einen Patienten.

7. Peptide nach Anspruch 6 in einer wirksamen Konzentration zur Verabreichung an einen Patienten zur Neutralisation zirkulierender Autoantikörper.

8. Peptide nach Anspruch 6 oder 7, die ferner einen pharmazeutischen Träger zur Verabreichung an einen Patienten umfassen, wobei der Träger und die Konzentration der Sequenzen bei Verabreichung an einen Wirt eine Immunantwort hervorrufen.

9. Peptide nach einem der Ansprüche 1-5, die mit einer Verbindung markiert sind, die aus Farbstoffen, fluoreszierenden Markierungen, chemilumineszierenden Markierungen, Enzymen und radioaktiven Markierungen ausgewählt ist.

10. Peptide nach einem der Ansprüche 1-5, die an ein Substrat immobilisiert sind.

11. Ex-vivo-Verfahren zum Screenen von Patienten auf Autoimmunstörungen durch Umsetzen einer aus einem Patienten entnommenen biologischen Probe mit einem Peptid nach einem der Ansprüche 1-5.

12. Verfahren nach Anspruch 11, das des weiteren ein Nachweisen von Autoantikörpern in der Probe des Patienten umfaßt.

13. Verfahren nach Anspruch 12, das des weiteren eine Krankheitsvorhersage des Patienten auf der Basis der Reaktivität der Probe des Patienten mit den Peptiden umfaßt.

14. Peptide nach einem der Ansprüche 1-10 zur Verwendung in der Medizin.

15. Verwendung eines Peptids nach einem der Ansprüche 1-10 bei der Herstellung eines Medikaments zur Verwendung in einem verfahren zur Behandlung von Patienten bezüglich Autoimmunstörungen durch Verabreichen des Medikaments an den Patienten.

16. Verwendung nach Anspruch 15, wobei das Peptid als Vaccine gegen eine Autoimmunstörung wirkt.

17. Verwendung nach Anspruch 15, wobei das Peptid an die Autoantikörper bindet, um die Autoimmunantwort zu blockieren.

18. Verwendung nach Anspruch 15, wobei die Peptide in Kombination verabreicht werden, um eine mehr als einen Autoantikörper umfassende Autoimmunantwort zu blockieren.

## Revendications

1. Peptide formant un épitope linéaire pour un auto-anticorps humain choisi soit (i) dans le groupe de peptides ayant moins de quarante acides aminés, où la séquence de l'épitope commence avec l'acide aminé numéroté à partir de l'extrémité terminale amino suivie de la séquence d'acides aminés indiquée constituée par
les épitopes de La/SSB :
ICHQIEYYFGDFNLPRDKFLK (ID N° 20 du listage des séquences),
YFGDFNLP (acides aminés 8 à 15 de la ID N° 20 du listage des séquences),
WVPLEIMIKFNR (ID N° 21 du listage des séquences),
VPLEIMIK (acides aminés 2 à 9 de la ID N° 21 du listage des séquences),
NRLNRLTTDFNVIVE (ID N° 23 du listage des séquences),
NRLNRLTT (acides aminés 1 à 8 de la ID N° 23 du listage des séquences),
TDFNVIVE (ID N° 30 du listage des séquences),
DFNVIVEA (acides aminés 2 à 9 de la ID N° 30 du listage des séquences),
KTKIRRSPSKPL (ID N° 22 du listage des séquences),
KIRRSPSK (acides aminés 4 à 11 de la ID N° 22 du listage des séquences),
YKNDVKNRSVYIKGFPT (ID N° 29 du listage des séquences),
SVYIKGFP (acides aminés 9 à 16 de la ID N° 29 du listage des séquences),
QVLNIQMRRTLHKAFKGS (ID N° 19 du listage des séquences),
NIQMRRTLHKAFK (acides aminés 4 à 16 de la ID N° 19 du listage des séquences),
RTLHKAFK (acides aminés 9 à 16 de la ID N° 19 du listage des séquences),
IFVVFDSIE (ID N° 27 du listage des séquences),
FVVFDSIE (acides aminés 2 à 9 de la ID N° 27 du listage des séquences),
KETDLLILFKDDYFA (ID N° 28 du listage des séquences),
ILFKDDYF (acides aminés 7 à 14 de la ID N° 28 du listage des séquences),
KVEAKLRAKQ (ID N° 36 du listage des séquences),
EAKLRAKQ (acides aminés N° 3 à 10 de la ID N° 36 du listage des séquences),
CLLKFSGD (ID N° 34 du listage des séquences),
REDLHILF (ID N° 33 du listage des séquences),
GEIKWIDFVRGAK (ID N° 24 du listage des séquences),
KWIDFVRGAK (acides aminés 4 à 13 de la ID N° 24 du listage des séquences),
IDFVRGAK (acides aminés 6 à 13 de la ID N° 24 du listage des séquences),
EGIILFKEKAK (ID N° 31 du listage des séquences),
EGIILFKE (acides aminés 1 à 8 de la ID N° 31 du listage des séquences),
GNLQLRNKEVTW (ID N° 26 du listage des séquences),
LRNKEVTW (acides aminés 5 à 12 de la ID N° 26 du listage des séquences),
SLNKWKSKGRRFKGKGKGNK (ID N° 25 du listage des séquences),
KSKGRRFK (acides aminés 6 à 13 de la ID N° 25 du listage des séquences),
KVQFQGKKTKFASD (ID N° 32 du listage des séquences),
KKTKFASD (acides aminés 7 à 14 de la ID N° 32 du listage des séquences),
TGPVKRAR (ID N° 35 du listage des séquences),
les épitopes de Ro/SSA :
MNRLHRFL (ID N° 37 du listage des séquences),
LCFGSEGGT (ID N° 38 du listage des séquences),
CFGSEGGT (acides aminés 2 à 9 de la ID N° 38 du listage des séquences),
SEGGTYYIKEQ (ID N° 39 du listage des séquences),
EGGTYYIKEQ (acides aminés 2 à 11 de la ID N° 39 du listage des séquences),
GTYYIKEQ (acides aminés 4 à 11 de la ID N° 39 du listage des séquences),
GTYYI (acides aminés 4 à 8 de la ID N° 39 du listage des séquences),
EIKSFSQEGRT (ID N° 40 du listage des séquences),
KSFSQEGR (acides aminés 3 à 10 de la ID N° 40 du listage des séquences),
SQEGRTTKQ (ID N° 41 du listage des séquences),
GRTTKQEPM (ID N° 42 du listage des séquences),
STKQAAFKAV (acides aminés 2 à 11 de la ID N° 43 du listage des séquences),
ISTKQAAFKAVS (ID N° 43 du listage des séquences),
KQAAFKAV (acides aminés 4 à 11 de la ID N° 43 du listage des séquences),
AFKAVSEVC (ID N° 44 du listage des séquences),
FTFIQFKKDLKESMK (ID N° 45 du listage des séquences),
QFKKDLKE (acides aminés 5 à 12 de la ID N° 45 du listage des séquences),
SMKCGMWGRA (ID N° 46 du listage des séquences),
MKCGMWGRA (acides aminés 2 à 10 de la ID N° 46 du listage des séquences),
GMWGRALRKAIA (ID N° 47 du listage des séquences),
GRALRKAI (acides aminés 4 à 11 de la ID N° 47 du listage des séquences),
ALAVTKYKQRNGWSHKDLLRLSH (ID N° 48 du listage des séquences),
TKYKQRNG (acides aminés 5 à 12 de la ID N° 48 du listage des séquences),
QRNGWSHK (acides aminés 9 à 16 de la ID N° 48 du listage des séquences),
LLRLSHLKPSS (ID N° 49 du listage des séquences),
RLSHLKPS (acides aminés 3 à 10 de la ID N° 49 du listage des séquences),
TKYITKGW (acides aminés 2 à 9 de la ID N° 71 du listage des séquences),
ITKGWKEV (acides aminés 5 à 12 de la ID N° 71 du listage des séquences),
HELYKEKA (ID N° 50 du listage des séquences),
LYKEKALSV (ID N° 51 du listage des séquences),
KALSVETEKLLKYL (ID N° 52 du listage des séquences),
TEKLLKYL (acides aminés 7 à 14 de la ID N° 52 du listage des séquences),
KLLKYLEA (ID N° 53 du listage des séquences),
LEAVEKVKRTKDE (ID N° 54 du listage des séquences),
KVKRTKDE (acides aminés 6 à 13 de la ID N° 54 du listage des séquences),
HLLTNHLKSKEVWKALLQEMPL (ID N° 55 du listage des séquences),
LKSKEVWK (acides aminés 7 à 14 de la ID N° 55 du listage des séquences),
KSKEVWKA (acides aminés 8 à 15 de la ID N° 55 du listage des séquences),
SKEVWK (acides aminés 9 à 14 de la ID N° 55 du listage des séquences),
ALLRNLGKMTA (ID N° 56 du listage des séquences),
RNLGKMT (acides aminés 4 à 10 de la ID N° 56 du listage des séquences),
LGKMTANS (ID N° 57 du listage des séquences),
LCNEKLLKKARIHPFHI (ID N° 58 du listage des séquences),
LLKKARI (acides aminés 6 à 12 de la ID N° 58 du listage des séquences),
KKARIHPF (acides aminés 8 à 15 de la ID N° 58 du listage des séquences),
TYKTGHGLRGKLKWRPDE (ID N° 59 du listage des séquences),
YKTGHGL (acides aminés 2 à 8 de la ID N° 59 du listage des séquences),
ALDAAFYK (ID N° 60 du listage des séquences),
AAFYKTFKTVEPTGKRFLLA (ID N° 61 du listage des séquences),
ASMNQRVLGS (ID N° 62 du listage des séquences),
EPTGKRFL (acides aminés 11 à 18 de la ID N° 61 du listage des séquences),
AMCMVVTR (ID N° 63 du listage des séquences),
AFSDEMVP (ID N° 64 du listage des séquences),
VPCPVTTD (ID N° 65 du listage des séquences),
VLMAMSQI (ID N° 66 du listage des séquences),
TDCSLPMI (ID N° 67 du listage des séquences),
PLMIWAQKTNTPA (acides aminés 3 à 15 de la ID N° 68 du listage des séquences),
TFAGGVHPAI (ID N° 69 du listage des séquences),
TFAGGVHP (acides aminés 1 à 8 de la ID N° 69 du listage des séquences),
IALREYRKKMDIPAKL (ID N° 70 du listage des séquences),
REYRKKMD (acides aminés 4 à 11 de la ID N° 70 du listage des séquences),
les épitopes de nRNP de 70 kD :
ALFAPRDP (ID N° 72 du listage des séquences),
ERMERKRR (ID N° 73 du listage des séquences),
MVYSKRSG (ID N° 74 du listage des séquences),
GKKIDGRR (ID N° 75 du listage des séquences),
VERGRTVK (ID N° 76 du listage des séquences),
VKGWRPRR (ID N° 77 du listage des séquences),
RRSRSRDK (ID N° 78 du listage des séquences),
RRRSRERS (ID N° 79 du listage des séquences),
SRERSKDK (ID N° 80 du listage des séquences),
KRRSSRSR (ID N° 81 du listage des séquences), et
RRSHRSER (ID N° 82 du listage des séquences) ;
les épitopes de peptide A de nRNP :
LNEKIKKD (ID N° 83 du listage des séquences),
KKDELKKS (ID N° 84 du listage des séquences),
SRSLKMRG (ID N° 85 du listage des séquences),
PFYDKPMR (ID N° 86 du listage des séquences),
IIAKMFGTF (ID N° 87 du listage des séquences),
DRKREKRK (ID N° 88 du listage des séquences),
QETPATKK (ID N° 89 du listage des séquences),
ALQGFKIT (ID N° 90 du listage des séquences), et
MKISFAKK (ID N° 91 du listage des séquences) ; et
les épitopes de peptide C de nRNP :
RKHKENVK (ID N° 92 du listage des séquences),
KDYYQKWN (ID N° 93 du listage des séquences),
AFQQGKIP (ID N° 94 du listage des séquences),
KIPPTPFS (ID N° 95 du listage des séquences),
PPPPSLPG (ID N° 96 du listage des séquences),
SLPGPPRP (ID N° 97 du listage des séquences),
PPRPGMMP (ID N° 98 du listage des séquences),
PPPPGMMP (ID N° 99 du listage des séquences),
GPAPGMRP (ID N° 100 du listage des séquences),
PPMMRPPA (ID N° 101 du listage des séquences), et
PGMTRPDR (ID N° 102 du listage des séquences) ;
soit (ii) un groupe de peptides tel que défini en (i) constitué de quatre à vingt acides aminés, mais ne comprenant pas les peptides ayant une séquence d'acides aminés constituée par A I A L R E Y R K K M D I P A, V H P A I A L R, K L G L E N, K D L K, K S K E, L T A L L R, R G K L K W, L K A L D, T R T E K D S, D E M V, E Y R K K M D, A A A M, D P D D, L S H L. K, R T K D E, E K D S, E V C R, ni les peptides correspondant aux résidus 1 à 11, 35 à 58, 56 à 77, 74, 96, 131 à 153, 149 à 166, 163 à 184, 180 à 205, 203 à 225 et 257 à 282 dans la séquence d'un UIA de Sn RNP ; et
où les peptides constitués de quatre à sept acides aminés sont contenus à l'intérieur des séquences d'acides aminés définis en (i) en tant que début de l'épitope.

2. Peptide du groupe (i) selon la revendication 1.

3. Peptide du groupe (ii) selon la revendication 1, constitué de six à vingt-cinq acides aminés.

4. Peptides selon la revendication 1, réactifs avec des anticorps polyclonaux anti-Ro/SSA ou des anticorps polyclonaux anti-La/SSB.

5. Peptides selon la revendication 1, dans un véhicule pharmaceutiquement acceptable et en une quantité efficace pour déclencher un effet immunogène chez un humain, ou bloquer des auto-anticorps, où le peptide est choisi dans l'ensemble constitué par :
GTFKAFDK (ID N° 1 du listage des séquences),
TFKAFDKHM (ID N° 15 du listage des séquences),
CDEFRKIKPKNAKQP (ID N° 2 du listage des séquences),
EGPPPKDT (ID N° 16 du listage des séquences),
KDTGIARV (ID N° 17 du listage des séquences), et
RVPLAGAA (ID N° 3 du listage des séquences),
AGGPGVGRAAGRGVPAG (ID N° 4 du listage des séquences),
IPQAPAGLAG (ID N° 18 du listage des séquences),
AGLAGPVRGVGGPSQ (ID N° 5 du listage des séquences),
QVMTPQGRGTVA (ID N° 6 du listage des séquences),
PTQYPPGRGTPPPPV (ID N° 7 du listage des séquences),
TPPPPVGRATPPPGI (ID N° 8 du listage des séquences),
PPPGIMAP (ID N° 9 du listage des séquences),
MAPPPGMRPPM (ID N° 10 du listage des séquences),
PIGLPPARGTPIGMPP (ID N° 11 du listage des séquences),
PIGMPPPG (ID N° 12 du listage des séquences),
RPPPPGIRGPP (ID N° 13 du listage des séquences),
RGPPPPGMRPPR (ID N° 14 du listage des séquences),
ou leurs mélanges.

6. Peptides selon l'une quelconque des revendications 1 à 4, en combinaison avec un véhicule pharmaceutique pour une administration à un patient.

7. Peptides selon la revendication 6, en une concentration efficace pour une administration à un patient afin de neutraliser les auto-anticorps en circulation.

8. Peptides selon la revendication 6 ou 7, comprenant en outre un véhicule pharmaceutique pour une administration à un patient, où le véhicule et la concentration de séquences déclenchent une réponse immunitaire lors d'une administration à un hôte.

9. Peptides selon l'une quelconque des revendications 1 à 5, marqués avec un composé choisi dans l'ensemble constitué par les colorants, les marqueurs fluorescents, les marqueurs chimioluminescents, les enzymes, et les marqueurs radioactifs.

10. Peptides selon l'une quelconque des revendications 1 à 5, immobilisés sur un substrat.

11. Procédé ex vivo pour dépister des maladies auto-immunes chez des patients, comprenant la réaction d'un échantillon biologique soutiré du patient avec un peptide tel que revendiqué dans l'une quelconque des revendications 1 à 5.

12. Procédé selon la revendication 11, comprenant en outre la détection d'auto-anticorps dans l'échantillon du patient.

13. Procédé selon la revendication 12, comprenant en outre la prédiction du pronostic du patient sur la base de la réactivité de l'échantillon du patient avec les peptides.

14. Peptide selon l'une quelconque des revendications 1 à 10, à utiliser en médecine.

15. Utilisation d'un peptide selon l'une quelconque des revendications 1 à 10 dans la fabrication d'un médicament à utiliser dans un procédé pour traiter des troubles auto-immuns chez des patients, comprenant l'administration du médicament au patient.

16. Utilisation selon la revendication 15, dans laquelle le peptide agit comme un vaccin contre un trouble auto-immun.

17. Utilisation selon la revendication 15, dans laquelle le peptide se fixe aux auto-anticorps pour bloquer la réponse auto-immune.

18. Utilisation selon la revendication 15, dans laquelle les peptides sont administrés en combinaison pour bloquer une réponse auto-immune mettant en jeu plus d'un auto-anticorps.
